# EUROPEAN PATENT SPECIFICATION

(11) **EP 1 184 392 B1**
(45) Date of publication and mention of the grant of the patent: **04.04.2007**
(21) Application number: 00929885.2
(22) Date of filing: 26.05.2000
(51) Int. Cl.: C07K 14/775, G01N 33/92, C07K 16/18

(54) **STABILIZED DENATURED LIPOPROTEIN AND PROCESS FOR PRODUCING THE SAME**
STABILISIERTES DENATURIERTES LIPOPROTEIN UND VERFAHREN ZU DESSEN HERSTELLUNG
LIPOPROTEINE DENATUREE STABILISEE ET PROCEDE DE PRODUCTION DE CETTE LIPOPROTEINE

(30) Priority: 02.06.1999 JP 15519899
(43) Date of publication of application: 06.03.2002
(73) Proprietor: KYOWA MEDEX CO., LTD., Tokyo 104-0042 (JP)
(72) Inventor: SHIGEMATSU, Takashi , Vessel Res. Lab. Co., Ltd., Machida-shi, Tokyo 194-8533 (JP); SHIMAMURA, Kyoko, Vessel Res. Lab. Co., Ltd., Machida-shi, Tokyo 194-8533 (JP); KIMURA, Junji, Vessel Res. Lab. Co., Ltd., Machida-shi, Tokyo 194-8533 (JP); KOHNO, Hiroaki, Head Office, Kyowa Medex Co., Ltd., Tokyo 104-0042 (JP); SUESHIGE, Nobuyuki, Kyowa Medex Co., Ltd., Sunto-gun, Shizuoka 411-0932 (JP)
(74) Representative: Lee, Nicholas John
(86) International application number: PCT/JP2000/003413
(87) International publication number: WO 2000/075189

(56) References cited:
- EP-A- 0 617 289
- EP-A1- 0 663 407
- EP-A3- 0 617 289
- JP-A- 9 297 137
- SCHIELE E ET AL: "FEASIBILITY OF A RECOMBINANT HUMAN APOLIPROTEIN E REFERENCE MATERIAL" FRESENIUS JOURNAL OF ANALYTICAL CHEMISTRY, SPRINGER, BERLIN, DE, vol. 360, 1998, pages 501-504, XP002931589 ISSN: 0937-0633
- COSTANTINO H.R. ET AL: "Moisture-induced aggregation of lyophilized insulin" PHARMACEUTICAL RESEARCH, vol. 11, 1994, pages 21-29, XP001078784
- PATENT ABSTRACTS OF JAPAN vol. 1998, no. 03, 27 February 1998 (1998-02-27) & JP 09 297137 A (SHINOTESUTO:KK), 18 November 1997 (1997-11-18)
- ITABE H ET AL: "A MONOCLONAL ANTIBODY AGAINST OXIDIZED LIPOPROTEIN RECOGNIZES FOAM CELLS IN ATHEROSCLEROTIC LESIONS COMPLEX FORMATION OF OXIDIZED PHOSPHATIDYLCHOLINES AND POLYPEPTIDES" JOURNAL OF BIOLOGICAL CHEMISTRY, AMERICAN SOCIETY OF BIOLOGICAL CHEMISTS, BALTIMORE, MD, US, vol. 269, no. 21, 27 May 1994 (1994-05-27), pages 15274-15279, XP002944332 ISSN: 0021-9258
- MURAKAMI M. ET AL.: 'Distinction in the mode of receptor-mediated endocytosis between high density lipoprotein and acetylated high density lipoprotein: evidence for high density lipoprotein receptor-mediated cholesterol transfer' J. BIOCHEM. vol. 101, no. 3, 1987, pages 729 - 741, XP002931588
- SCHIELE F. ET AL.: 'Feasibility of a recombinant human apolipoprotein E refenrece material' FRESENIUS J. ANAL. CHEM. vol. 360, no. 3/4, 1998, pages 501 - 504, XP002931589

## Description

### Technical Field

The present invention relates to stabilized denatured lipoprotein and a method for the production thereof. More particularly, the present invention relates to a method for the production of denatured lipoprotein, comprising the steps of: freezing a solution containing lipoprotein to produce a frozen solution; and melting the frozen solution to produce a solution containing denatured lipoprotein.

More particularly, the present invention relates to a method for the production of denatured lipoprotein described above, further comprising freeze-drying the solution containing denatured lipoprotein.

The denatured lipoprotein strongly suggests the association thereof with various diseases of the circulatory system including such diseases of the coronary artery system as cardiac infarction and stenocardia, such diseases of the cerebral arteries as cerebral infarction and cerebrovascular dementia, such diseases of the renal arteries as nephropathy and diabetic nephropathy, and such diseases of the peripheral artery system as obstruction of peripheral arteries. The standard substances for determining mass of denatured lipoprotein and the various experimental reagents for the investigation of physiological role and physiological activity of the denatured lipoprotein, therefore, constitute themselves very important substances that affect the results of the research efforts. The denatured lipoprotein which has been stabilized in the manner described above, therefore, is useful as a standard substance in a method for determining denatured lipoprotein content in a blood component as by causing the denatured lipoprotein to contact an antibody capable of recognizing the denatured lipoprotein and determining the reactivity of the antibody with a sample and as a varying experimental reagent for investigating the physiological role and physiological activity of the denatured lipoprotein.

### Background Art

In various diseases of the circulatory system including such diseases of the coronary artery system as cardiac infarction and stenocardia, such diseases of the cerebral arteries as cerebral infarction and cerebrovascular dementia, such diseases of the renal arteries as nephropathy and diabetic nephropathy, and such diseases of the peripheral artery system as obstruction of peripheral arteries, it is strongly suggested that the lipid in the blood serum plays an important role. A huge amount of expenses are being paid today by the public health system for medical treatments using serum lipid depressants, particularly cholesterol depressants. Recent studies have yielded a report in which a comparison between a group of patients of such diseases and a group of healthy persons reveals no noticeable difference in the absolute amount of serum lipid between the two groups and that rather the amount of oxidized LDL, a denatured product of low-density lipoprotein (LDL), present in the blood serum is distinctly different between the two groups (Toshima, S. Et al. (1996) Circulation, 94, Suppl. I: 1288). The relationship between the oxidized lipoprotein, one of the denatured lipoproteins, and the advance of atherosclerotic lesion has been pointed out by Steinberg et al. (Steinberg, D., Parthasarathy, S. Carew, T. E., Khoo, J. C., and Witztum, J. L., (1989) N. Engl. J. Med., 320: 915) for example. In recent years, therefore, various methods for determination using the denatured lipoprotein have been under development (Patent publication JP-A-08-304,395 and Patent publication JP-A-09-288,106, for example) and the test for investigating the physiological role of the denatured lipoprotein has been gaining in importance.

The fact that it is difficult to obtain the standard substances necessary for the determinations performed in such experiments, however, has complicated the situation. For the purpose of investigating the physiological role of denatured lipoprotein, it becomes necessary to collect a large number of blood samples of denatured lipoprotein from a plurality of installations and compare them. For the comparison, it is essential that the measurements obtained in the individual tests be free from variation. No reproducibility can be ensured among these measurements unless a standard substance stable and excellent in reproducibility throughout a duration necessary for a series of relevant tests is available. When the test results are markedly varied among different persons who measure owing to the use of different standard substances, the interpretation of the physiological role of given denatured lipoprotein is so complicated as to render it impossible to obtain a fixed conclusion. This inability to obtain a standard substance which fits stable preservation has closed the way of applying the determination of the amount of denatured lipoprotein present in a given sample, for example, as a means to judge a disease exactly despite popular recognition of the physiological importance of this determination.

Generally, in the determination of the mass of protein contained in blood serum, for example, the practice of stabilizing by some method or other what may be called a standard blood serum or a target component in an isolated state and using the product of this stabilization as a standard substance is now carried out. As regards the lipoprotein in general, a method for producing standard blood plasma or standard blood serum stable during prolonged preservation by optionally mixing lipoprotein-containing blood plasma or blood serum with such nonreducing sugar as sucrose and freeze-drying the resultant mixture till the water content reaches a level in the range of 1 - 10 mass % (Patent publication EP-A-617289) and a method for commercially producing a stable freeze-dried product by obtaining reconstructive lipoprotein from apolipoprotein and lipid and freeze-drying and stabilizing the reconstructive lipoprotein in the presence of a stabilizer such as sucrose or mannitol (Patent publication US-A-5,652,399) have been reported. The patents published in these official gazettes merely attempt to stabilize lipoprotein as a means to stabilize the lipoprotein without entailing denaturation thereof and do not disclose the stabilized denatured lipoprotein and a method for the production thereof.

A method for obtaining such denatured lipoprotein as oxidized lipoprotein, acetylated lipoprotein, or malondialdehyde-conjugated lipoprotein by isolating and purifying lipoprotein by a heretofore well-known technique for example, ultracentrifugation and then oxidizing the refined lipoprotein with such a metal ion as a copper ion, acetylating it by the reaction with acetic anhydride, or allowing it to react with malondialdehyde has been known. The denatured lipoproteins produced by this method, however, are more unstable than the undenatured lipoprotein and unable in their denatured state to allow prolonged preservation.

In view of this state of affairs, Patent publication JP-A-09-288,106 has disclosed a method for determining human oxidized lipoprotein by using as a standard what is produced by incorporating into blood plasma lipoprotein the oxide of phospholipid obtained by artificially oxidizing phospholipid. The standard substance which is used in the method described above, however, is so deficient in stability of preservation as to require the individual preparation thereof prior to each use and entail a complicated procedure.

In view of such various states of affairs, the development of denatured lipoprotein capable of being stably stored for a long time and a method for the production thereof has been desired for.

Therefore objects of the present invention are to provide denatured lipoprotein excelling in stability of prolonged preservation, namely, the lipoprotein does not cause any discernible variation in the determinations throughout the duration of preservation and a method of the production thereof, wherein the lipoprotein is applied as a standard substance for the determination of the mass of denatured lipoprotein contained in a given blood component or as a varying experimental reagent for the investigation of physiological role of denatured lipoprotein.

### Disclosure of the Invention

In a first aspect, the invention provides a method for the production of denatured lipoprotein, comprising the steps of: freezing a solution containing lipoprotein to produce a frozen solution; and melting the frozen solution to produce a solution containing denatured lipoprotein.

In a second aspect, the invention provides stabilized denatured lipoprotein produced by a method of the first aspect.

In a third aspect, the invention provides a method for the determination of denatured lipoprotein in a sample, comprising the steps of: selecting a stabilized denatured lipoprotein according to the second aspect as a standard substance; forming a calibration curve using prescribed concentrations of the standard substance; reacting the sample with an antibody which binds to the denatured lipoprotein; and measuring the reactivity of the antibody with the sample.

In a fourth aspect, the invention provides a reagent kit for the determination of denatured lipoprotein, comprising stabilized denatured lipoprotein according to the second aspect as a standard substance. The reagent kit may comprise a diluting liquid for a sample, a solid phase formed by immobilizing an antibody, a reaction buffer, a washing solution, a labelled secondary antibody, a detecting reagent, and the whole or part of stabilized denatured lipoprotein according to the second aspect as a standard substance as component elements.

Particular embodiments of each aspect of the invention are set out in the dependent claims.

The present inventors, after a diligent study pursued with a view to accomplishing the object mentioned above, have found that by performing a process including at least one freezing operation on a solution containing lipoprotein thereby denaturing the lipoprotein contained in the solution, it is made possible to obtain a standard substance usable for the determination of a denatured protein content in a given blood sample or a varying experimental reagent usable for the investigation of physiological role or physiological activity of the denatured protein. They have further found that by freeze-drying the denatured lipoprotein obtained as described above, it is made possible to improve prominently the stability of prolonged preservation of the denatured lipoprotein in the dried state and the stability of preservation of the denatured lipoprotein in the dried state after it has been dissolved into a solution. The present inventors have further found that by allowing the presence of sucrose, lactose, trehalose, bovine blood serum albumin (BSA), or human blood serum albumin (HSA) etc. as a stabilizer during the course of freeze-drying, it is made possible to improve the denatured lipoproteins to a greater extent in stability of prolonged preservation and give a better solution to the problems mentioned above.

The present invention has been perfected based on such knowledge as mentioned above.

The object mentioned above is accomplished by a method for producing denatured lipoprotein by performing a process including at least one freezing operation on a solution containing lipoprotein, thereby denaturing the lipoprotein contained in the solution.

The object is further accomplished by a method for producing stabilized denatured lipoprotein, which comprises performing a process including at least one freezing operation on a solution containing lipoprotein, thereby denaturing the lipoprotein contained in the solution and obtaining denatured lipoprotein and further freeze-drying the denatured lipoprotein, thereby stabilizing the denatured lipoprotein.

### Brief Description of the Drawings

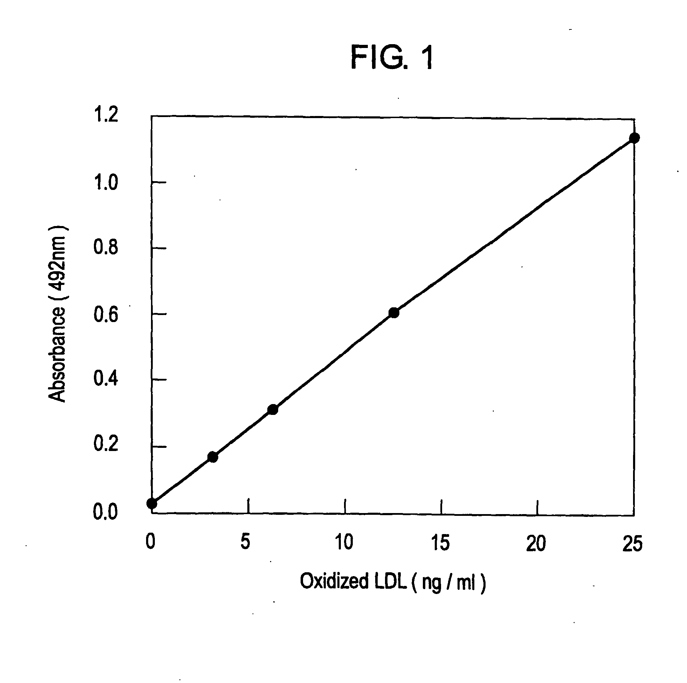

Fig. 1 is a diagram showing the calibration curve produced with the oxidized LDL obtained in comparative Example 1 of the present invention as a sample, in which the oxidized LDL is prepared by freeze-drying the LDL oxidized with copper and then dissolving the dry LDL by a prescribed method.

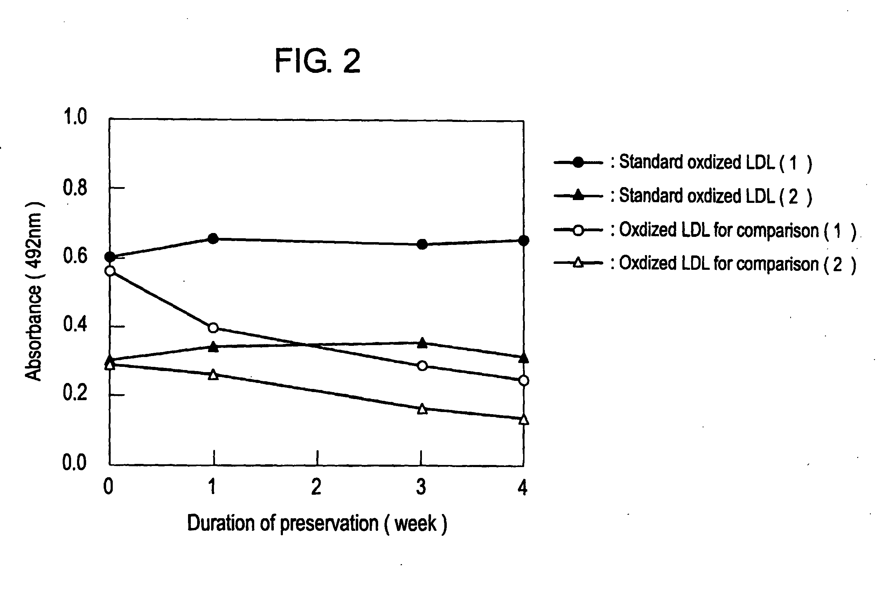

Fig. 2 is a diagram comparing the determinations obtained from the sample prepared by preserving the oxidized LDL obtained in comparative Example 1 of the present invention at 4°C in which the oxidized LDL is prepared by freeze-drying the LDL oxidized with copper ("Standard oxidized LDL"), and dissolving the LDL at stated durations by a prescribed method, and, the determinations at stated durations obtained from the sample prepared by preserving the LDL oxidized with copper at 4°C without being freeze-dried ("Oxidized LDL for comparison").

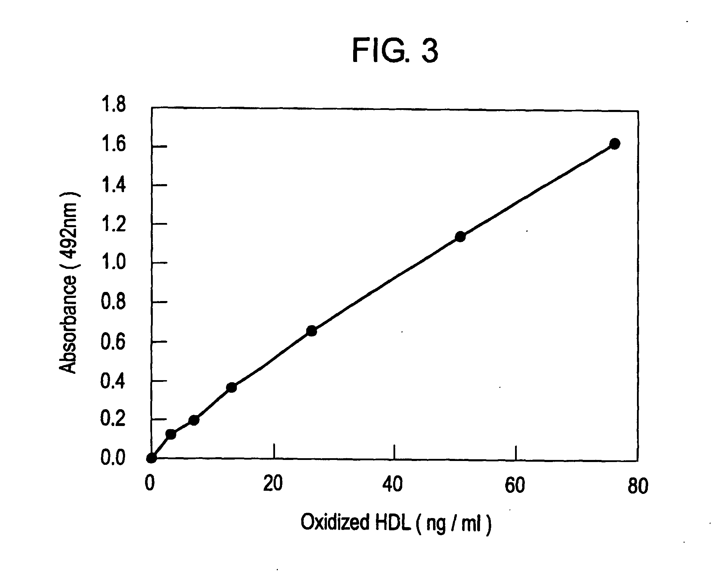

Fig. 3 is a diagram showing the calibration curve produced with the oxidized HDL obtained in comparative Example 2 of the present invention as a sample, in which the oxidized HDL is prepared by freeze-drying the HDL oxidized with copper and then dissolving the dried HDL by a prescribed method.

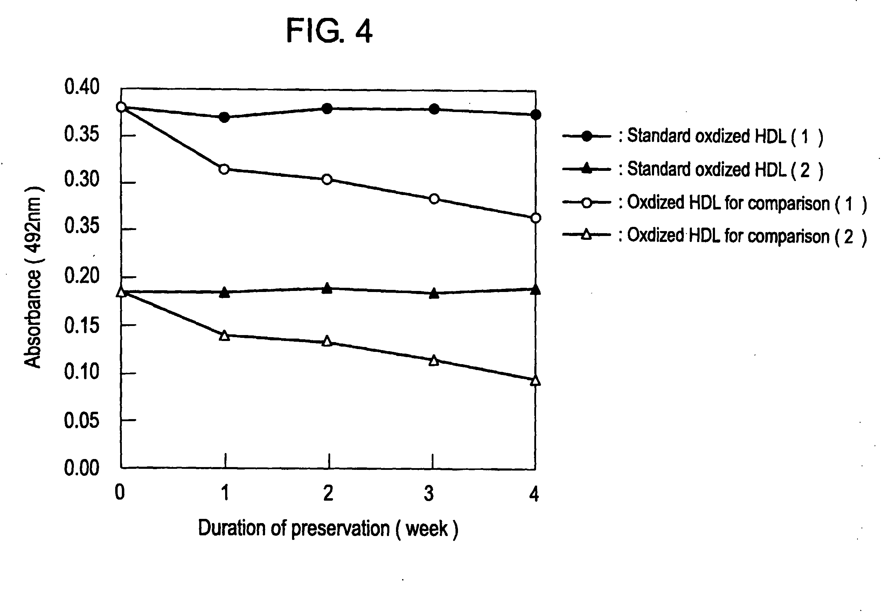

Fig. 4 is a diagram comparing the determinations obtained from the sample prepared by preserving the oxidized HDL obtained in comparative Example 2 of the present invention at 4°C in which the oxidized HDL is prepared by freeze-drying the HDL oxidized with copper ("Standard oxidized HDL"), and dissolving the HDL at stated durations by a prescribed method, and, the determinations at stated durations obtained from the sample prepared by preserving the HDL oxidized with copper at 4°C without being freeze-dried ("Oxidized HDL for comparison") .

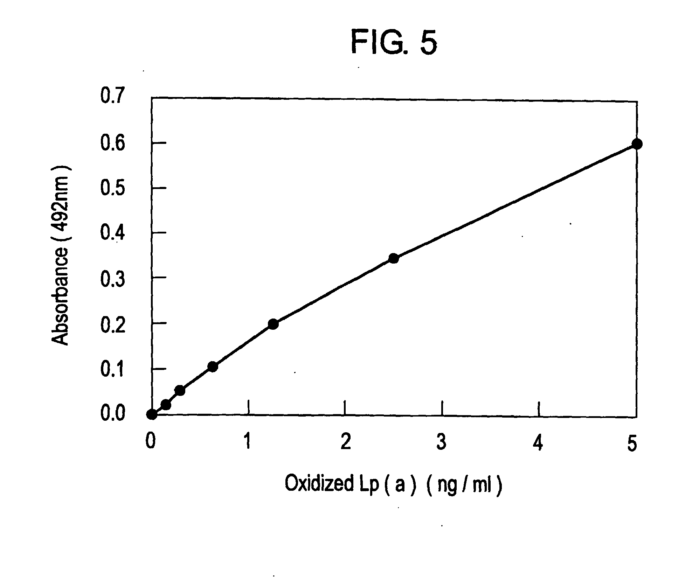

Fig. 5 is a diagram showing the calibration curve produced with the oxidized lipoprotein a [Lp (a)] obtained in comparative Example 3 of the present invention as a sample, in which the oxidized Lp(a) is prepared by freeze-drying the LDL oxidized with copper and then dissolving the dried Lp(a) by a prescribed method.

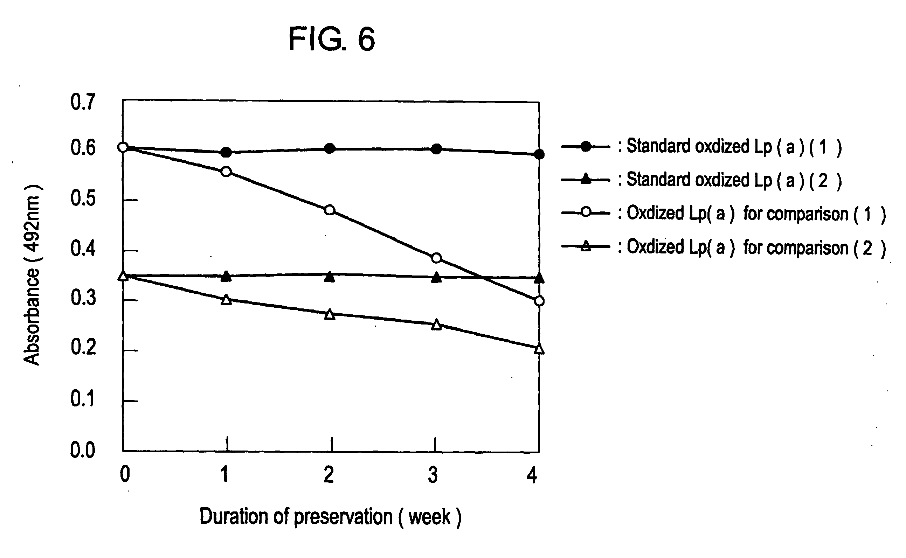

Fig. 6 is a diagram comparing the determinations obtained from the sample prepared by preserving the oxidized Lp(a) obtained in comparative Example 3 of the present invention at 4°C in which the oxidized Lp(a) is prepared by freeze-drying the Lp (a) oxidized with copper ("Standard oxidized Lp (a)"), and dissolving the Lp(a) at stated durations by a prescribed method, and, the determinations at stated durations obtained from the sample prepared by preserving the Lp(a) oxidized with copper at 4°C without being freeze-dried("Oxidized Lp (a) for comparison").

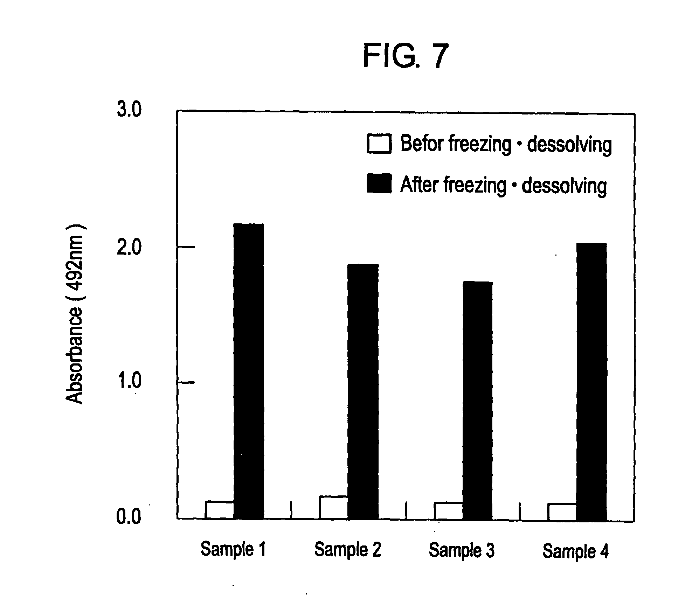

Fig. 7 is a diagram illustrating the production of denatured lipoprotein by performing a process including a step of freezing on blood plasma as demonstrated in Example 1.

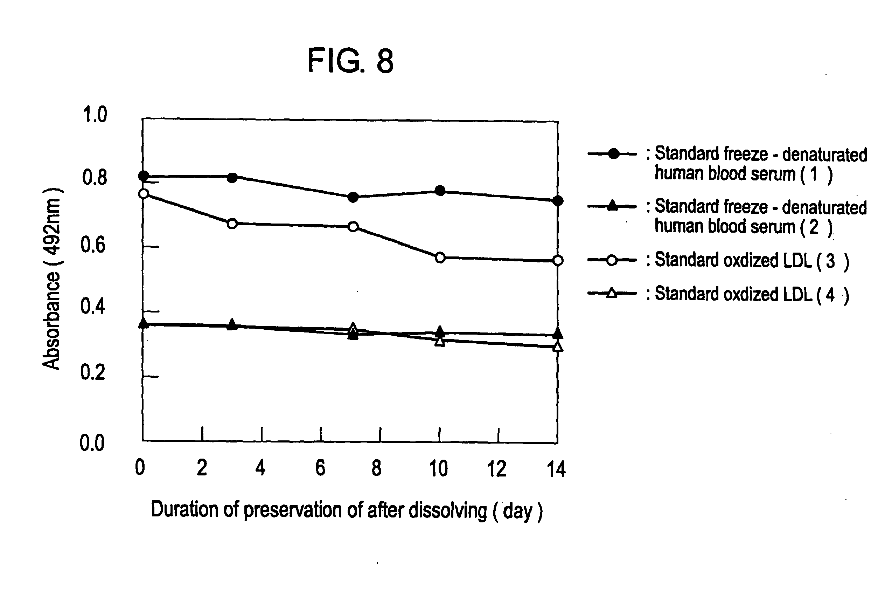

Fig. 8 is a diagram for comparing the determinations (absorbance) obtained in comparative Example 4 respectively of the samples of standard freeze-denatured human blood serum prepared in comparative Example 4 (2) and the samples of standard oxidized LDL prepared in comparative Example 1 (3) ("Standard oxidized LDL") after the samples had been dissolved and rated for stability of preservation according to the ELISA method.

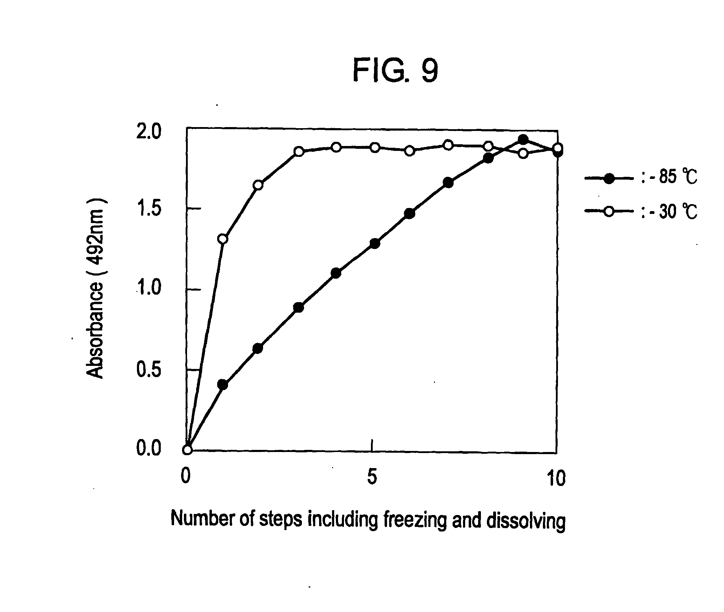

Fig. 9 is a diagram illustrating the production of denatured lipoprotein by performing a process including a freezing step on human LDL in Example 2.

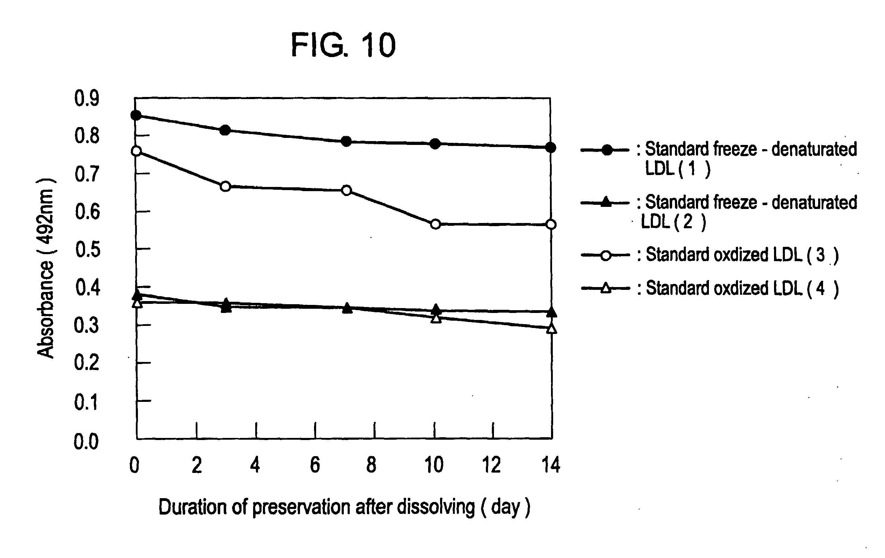

Fig. 10 is a diagram for comparing the determinations (absorbance) obtained in Example 2 respectively of the samples of standard freeze-denatured LDL prepared in Example 2 (2) and the samples of standard oxidized LDL prepared in comparative Example 1 (3) ("Standard oxidized LDL") after the samples had been dissolved and rated for stability of preservation according to the ELISA method.

Now, the present invention will be described in detail below.

The first aspect of the present invention consists in providing a method for producing stabilized denatured lipoprotein, comprising the steps of: freezing a solution containing lipoprotein to produce a frozen solution; and melting the frozen solution to produce a solution containing denatured lipoprotein.

The term "denatured lipoprotein" as used in this specification means both the denatured lipoprotein which has undergone such a chemical change as oxidized lipoprotein, acetylated lipoprotein and malondialdehyde-conjugated lipoprotein obtained by action of malondialdehyde etc., and the denatured lipoprotein which has undergone such a structural change as coagulation or change in three-dimensional structure and also includes such kinds of denatured lipoprotein as identified by a change in electric charge, a change in molecular weight, a change in affinity for an in vivo receptor, and a change in ability to form linkage with denatured lipoprotein specific antibody (J. Biol. Chem. 1994, 269: 15274-15279 and Patent publication JP-A-07-238,098) represented by the antibody yielded by FOHla/DLH3 (Deposit No.: FERM BP-7171) comparing to an undenatured lipoprotein. The expression "change in ability to form linkage with denatured lipoprotein specific antibody represented by the antibody yielded by FOH1a/DLH3" as mentioned above refers to the difference between the amount of signal obtained from an undenatured lipoprotein antigen and the amount of signal obtained from denatured lipoprotein antigen in an operation performed by transforming the undenatured lipoprotein and the denatured lipoprotein conforming to the present invention each as an antigen into an insolubilized solid phase, causing a relevant antibody to react with the solidified antigen, and detecting the amount of the antibody consequently bound to the solidified antigen as converted to the enzyme content by the use of an enzyme standardized antibody possessing specificity to the antibody.

The lipoprotein to be used in the present invention may originate in any of the living organisms. As concrete examples of the lipoprotein, the lipoproteins which originate in such mammals as man, cow, horse, goat, sheep, rabbit, dog, and guinea pig; such birds as fowl and quail; such fishes as salmon and herring; and such microorganisms as bacteria and mycetes may be cited. Further, as concrete examples of the lipoprotein to be used in the present invention, structural lipoproteins which occur in such biomembranes as cell membrane, mitochondrial cell, myelinic structure membrane, and bacterial cell membrane; soluble lipoproteins which occur in blood plasma, egg yolk, and milk; and such lipoprotein fractions obtained by fractionating these lipoproteins mentioned above by the ultracentrifugation technique to chylomicron, very low-density lipoprotein (VLDL), low-density lipoprotein (LDL), lipoprotein X, intermediate density lipoprotein (IDL), lipoprotein a [Lp(a)], high-density lipoproteins (HDL) like HDL2 and HDL3, and very high-density lipoprotein (VHDL) may be cited. These lipoproteins may be used either singly or in the form of a combination of two or more members. Of these lipoproteins, those of human origins are used preferably, chylomicron, VLDL, LDL, Lp(a), HDL2 or HDL3 originating in human blood plasma and blood serum, and mixtures thereof are used more preferably, and LDLs originating in human blood plasma and blood serum are used most preferably.

The human lipoprotein which is typically used in the present invention is prepared by separating a fraction with a prescribed specific gravity from human blood serum by such a universally known technique as centrifugal sedimentation or ultracentrifugation and purifying this fraction by such a heretofore known technique as dialysis or desalination. As concrete examples of the method for the preparation of the low-density lipoprotein (LDL), the following methods (1) - (3) may be cited.
(1) To 20-30mL of normal human blood serum, ethylenediamine sodium tetraacetate (EDTA-2Na) is added until a final concentration of 1 mmol/L. The resultant mixture is made to add NaBr with adjusting to a specific gravity of 1.000. The produced mixture is dispensed into centrifugal tubes, thereby obtained the portions of the mixture in the respective tubes. Buffer solutions is adjusted with NaBr to varied levels of specific gravity of 1.15, 1.063,1.019, and 1.006, and superposed respectively on the potions of mixture in sequence. Then the tubes contained the mixtures and the buffers are together centrifuged (120,000xg) at 4°C for 24 hours. The fractions consequently formed are sequentially separated from the upper to the down and measured for specific gravity with a refractometer to collect fractions having a specific gravity in the range of 1.019 - 1.063 as LDL fractions. The LDL fractions which have been obtained as described above are dialyzed, immediately after collection, with PBS [phosphate buffer of 10 mmol/L and NaCl (pH 7.4) of 140 mmol/L] including 0.25 mmol/L EDTA (Patent publication JP-A-07-238,098, paragraph No. 0040).
(2) To the heparinized human blood plasma, EDTA is added until a final concentration of 0.25 mmol/L. The blood plasma thus prepared is dispensed in a unit volume of 0.75 mL into centrifugation tubes (1 - 4mL). The portions of the blood plasma in the respective tubes and 0.15 mol/L NaCl of 250 µL containing 0.3 mmol/L EDTA and superposed in a unit volume of 250 µL on the portions are together centrifuged with 185,000xg at 10°C for 2.5 hours. 150 µL of the upper layer is discarded. 750 µL of the lower layer is taken and then made to add 150 µL of KBr solution (50 w/v %) until a specific gravity of 1.063. The blood plasma samples with the adjusted specific gravity are moved to the bottoms of the centrifugation tubes (1 - 4mL) and centrifuged with 244,000×g at 10°C for 16 hours. The orange band in the upper layer (about 100 - 150µL) is attentively collected and dialyzed against PBS containing 0.25 mmol/L EDTA at 4°C for 6 hours (3 liters of PBS replaced twice by two hours interval)(Patent publication JP-A-08-304,395, paragraph No. 0050); or
(3) From the human blood plasma obtained with EDTA as an anti-coagulating agent, the portion having specific gravity 1.019 - 1.063 is collected as a LDL fraction by the centrifugation technique. The LDL fraction, after having the purity thereof verified by the formation of a single sharp band on analysis by the agarose electrophoresis, is thoroughly dialyzed against a PBS solution (pH 7.4) containing 0.25 mmol/L EDTA (Patent publication JP-A-09-288,106, paragraph No.0062).

Then, as one concrete example of the method for preparing lipoprotein a [Lp(a)], for instance, the following method may be cited: To the heparinized human blood plasma, EDTA is added until a final concentration of 0.25 mmol/L. The blood plasma sample thus prepared and 250 µL of 0.15 mol/L NaCl containing 0.3 mmol/L EDTA and superposed on the sample are together centrifuged with 105,000xg at 8°C for 20 hours. The upper layer is discarded. In the lower layer, KBr pulverized by a mortar in advance is dissolved until a final specific gravity of 1.125 while avoiding formation of bubbles. The resultant solution is centrifuged with 105,000xg at 8°C for 20 hours. The orange band in the upper layer is collected attentively and then subjected to gel filtration with Biogel A-5m (Bio-rad Corp.) with 1 mol/L NaCl, 2 mmol/L EDTA, and 10 mmol/L phosphate buffer as a developing solvent. The fractions consequently obtained are each assayed with a Lp(a) measuring kit (produced by Thermo K.K.) to collect the Lp(a) fraction. This fraction is treated with lysine sepharose 4B (Farmacia Corp.). The fraction adsorbed thereon is eluted with a buffer containing 0.2 mol/L ε-aminocaproic acid and dialyzed against PBS containing 0.25 mmol/L EDTA (Patent publication JP-A-08-304,395, paragraph No. 0052).

As known concrete examples of the method for denaturing human lipoprotein which do not fall within the scope of the present invention, for instance, a method which consists in oxidizing the human lipoprotein in the presence of a metal ion, a method which consists in acetylating the human lipoprotein, a method which consists in incorporating aldehyde in the human lipoprotein by the use of malondialdehyde, and a method which consists in adding a solution dissolved a compound obtained by artificially oxidizing such lipoprotein as 1-palmitoyl-2-(9-oxononanoyl)-glycerol-3-phosphocholine and 1-palmitoyl-2-(5-oxovaleroyl)-glycerol-3-phosphocholine) in a proper solvent like DMSO to the human lipoprotein may be cited. In the methods enumerated above, the method which consists in oxidizing the human lipoprotein in the presence of a metal ion, the method which consists in acetylating the human lipoprotein, and the method which consists in having aldehyde incorporated in the human lipoprotein by the use of malondialdehyde are used favourably.

Now, the three preferred methods mentioned above will be described in detail below.

Firstly, the mode of oxidizing the human lipoprotein in the presence of a metal ion will be described in detail below. One example of the mode mentioned above will be described below: The human lipoprotein (fraction) which has been prepared as described above is deprived of EDTA by dialyzing against a buffer containing no EDTA [for example, PBS (pH 7.4)] and adjusted to a prescribed protein content (50 - 2000 µg/mL, preferably 100 - 500 µg/mL) . It is made to add copper sulfate (CuSO₄) until a prescribed concentration and then left reacting therewith at a temperature in the approximate range of 36 - 38°C.

As concrete examples of the metal ion to be used in the mode mentioned above, copper ions originating in copper (II) fluoride dihydrate, copper (II) bromide, copper (II) oxide, copper (II) hydroxide, copper (II) sulfate, copper (II) sulfate pentahydrate, copper (I) selenide, copper (II) selenide, copper (II) selenide pentahydrate, copper (II) hexafluorosilicate tetrahydrate, copper (II) acetate monohydrate, copper (II) tetraammine sulfate monohydrate, and copper (II) bis(ethylenediamine) sulfate dihydrate; iron ions originating in iron (II) chloride, iron (II) bromide hexahydrate, iron (II) nitrate hexahydrate, iron (II) thiocyanate trihydrate, iron (II) acetate tetrahydrate, iron (III) oxalate pentahydrate, iron (II) ammonium sulfate hexahydrate, iron (III) potassium sulfate dodecahydrate, iron (II) ammonium sulfate dodecahydrate, and iron sulfate; metal ions of hemoglobin (Hb), transferrin (Tf), and lactoferrin (Lf) and mixtures thereof may be cited. Among other metal ions enumerated above, the copper ions originating in copper (II) sulfate and copper (II) sulfate pentahydrate and mixtures thereof are preferably used. These metal ions may be used either singly or in the form of a combination of two or more members.

The amount of the metal ion to be used in the mode described above does not need to be particularly discriminated but is only required to permit thorough oxidation of the human lipoprotein. It may be such that the concentration of the metal ion will be in the range of 10 - 200 µmols/L, preferably in the range of 25 - 100 µmols/L, based on 1 g of the human lipoprotein to be oxidized.

In the mode described above, if the reaction time is unduly short, the shortage will be at a disadvantage in preventing the denaturation (oxidation) of lipoprotein from being effected sufficiently. Conversely, if the reaction time is unduly long, the excess will be at a disadvantage in forcing the lipoprotein itself to decompose excessively and fail into the loss of the antigenicity of the apoprotein, for example. Though the reaction time cannot be generally defined because it varies with the amount of the residual EDTA and the total amount of the solution, it is in the range of 1 - 24 hours, preferably in the range of 1-24 hours, preferably 2-4hours, at temperatures of about 36°C - 38°C in the case of the lipoprotein (fraction) prepared in the mode described above.

Secondly, the mode of acetylation of the human lipoprotein will be described in detail below. One example of this mode will be described below: The solution of the human lipoprotein (fraction) prepared in a prescribed protein concentration (about 500 - 2000 µg/mL, preferably 500-1000 µg/mL) as described above and saturated sodium acetate etc. added thereto in a volume equal thereto are stirred together at 0 °C - 4°C for 1 - 2 hours. Then, the resultant mixture and acetic anhydride added thereto in a volume in the range of 0.25 - 4 µL (namely 0.5 - 2 µL/mg, based on the amount of the human lipoprotein), preferably in the range of 0.4 - 2.4 µL (namely 0.8 - 1.2 µL/mg, based on the amount of the human lipoprotein) are stirred together at 0 °C - 4°C for 60 - 120 minutes. Then, the produced mixture is dialyzed thoroughly against PBS (pH 7.4) containing 0.25 mmol/L of EDTA of 500-1000 times as large in volume of the produced mixture, at 2 °C - 8°C, two or three times for not less than two hours/each.

Thirdly, the mode of the incorporation of aldehyde in the human lipoprotein by the use of malondialdehyde will be described in detail below. One example of the mode mentioned above will be described below: The solution of the human lipoprotein (fraction) prepared as described above in a prescribed protein concentration (about 0.25 - 1 mg/mL, preferably 0.25 - 0.5 mg/mL) in a 0.1 mol/L phosphate buffer (pH 6.5) and a malondialdehyde solution (obtained by thermally hydrolyzing 1 mol/L of malondialdehyde bisdimethyl acetal at 100°C for five minutes in the presence of 0.1 mol/L of hydrochlorid acid) added thereto in a volume in the range of 0.625 - 10 µL (namely 2.5 - 10 µL/mg, based on the amount of the human lipoprotein), preferably in the range of 1 - 6 µL (namely 4 - 6 µL/mg, based on the amount of the human lipoprotein) are left reacting with each other at 30 °C - 40°C for 2 - 4 hours. The resultant reaction solution is dialyzed thoroughly against PBS (pH 7.4) containing 0.25 mmol/L of EDTA of 500-1000 times as large in volume of the produced mixture, at 2°C - 8°C, two or three times for not less than two hours/each.

The method contemplated by the present invention may further include a step for freeze-drying the denatured lipoprotein obtained as described above for the purpose of stabilizing the product. In the present specification, the term "freeze-drying" is used in the meaning as used in the relevant field. Specifically, it means to freeze a given sample, decompress the sample as kept in a frozen state, and deprive the sample of water and a subliming component until it dries. The conditions for the freeze-drying in the present invention do not need to be particularly discriminated but are only required to be capable of stabilizing the denatured lipoprotein. The freeze-drying is generally effected at a temperature in the range of -80 °C - 20°C, preferably in the range of -80 °C - 15°C, under a pressure in the range of 0.667 - 13.33 Pa, preferably in the range of 0.667 - 1.333 Pa for a period in the range of 12 - 72 hours, preferably in the range of 24- 72 hours. By this step of freeze-drying, the water content in the freeze-dried product contained the denatured lipoprotein is generally made to fall to not more than 10 mass %, preferably to not more than 1 mass %.

In the present invention, the step of freeze-drying is preferred to proceed in the presence of a stabilizing agent. The stabilizing agent to be used in the mode of embodiment described above is the same stabilizing agent as used generally in the relevant field. As concrete examples of the stabilizing agent, saccharides such as sucrose, lactose, and trehalose; and proteins such as bovine blood serum albumin (BSA) and human blood serum albumin (HSA) may be cited. In these substances, sucrose, lactose, trehalose, bovine blood serum albumin (BSA), and human blood serum albumin (HSA) are preferably used as stabilizing agents. Incidentally, the stabilizing agents enumerated above may be used either singly or in the form of a mixture of two or more members. Though the amount of the stabilizing agent to be used does not need to be particularly restricted but is only required to be capable of stabilizing the denatured lipoprotein, it is generally in the range of 1 - 20 mass %, preferably in the range of 2 - 5 mass %.

In the present invention, the timing of the addition of a stabilizing agent during the course of the freeze-drying which is expected to proceed in the presence of the stabilizing agent does not need to be particularly restricted. The addition of the stabilizing agent preferably precedes the step of freeze-drying and particularly preferably intervenes between the step for denaturing and the step for freeze-drying. Further, the step for freeze-drying does not need to be particularly followed by any step for depriving the frozen lipoprotein of the stabilizing agent. In consideration of the stability of the preservation of the denatured lipoprotein, the continued presence of the stabilizing agent while the freeze-dried state is retained proves more preferable than not.

The present inventors have found that the denatured lipoprotein obtained by subjecting the solution containing lipoprotein to a process including at least one freezing operation thereby denaturing the lipoprotein contained in the solution is also usable as a standard substance for the purpose of determination of the mass of denatured lipoprotein in blood and as a reagent for investigating the physiological role and the physiological activity of the denatured lipoprotein and that when the denatured lipoprotein obtained as described above is further freeze-dried, the denatured lipoprotein excels in stability of prolonged preservation in the dried state and the denatured lipoprotein in the dried state, on being dissolved in a solution, enjoys stability of preservation. The methods of the aspect mentioned above have been conceived based on this knowledge.

The method of the first aspect of the present invention essentially requires to include a step for freezing the solution containing lipoprotein. As concrete examples of the solution containing lipoprotein, blood serum, blood plasma, and chylomicrons and such lipoprotein fractions as very low-density lipoprotein (VLDL), low-density lipoprotain (LDL), lipoprotein X, intermediate density lipoprotein (IDL), lipoprotein a [Lp(a)], high-density lipoproteins (HDL) like HDL2 and HDL3, and very high-density lipoprotein (VHDL) may be cited. In the solutions enumerated above, blood serum, blood plasma, chylomicrons, VLDL, LDL, Lp(a), HDL2, HDL3, and mixtures thereof are used more preferably and human blood plasma, human blood serum, and LSLs originating in the human blood plasma and human blood serum are used most preferably as solutions containing lipoprotein.

The method according to the first aspect of the present invention essentially requires to subject the solution containing lipoprotein to a process including a step for freezing. In the present specification, the term "process including at least one freezing operation" means a process which includes at least one step of freezing part or the whole of the water component in the lipoprotein contained in the solution or in the environment substantially encircling the lipoprotein. The denatured lipoprotein which is obtained by carrying out the process including the step for freezing as contemplated by the present invention may be any of the denatured lipoproteins enumerated above. The denatured lipoprotein which is obtained by carrying out the process including the step for freezing according to the present invention may be oxidized lipoprotein, lipoprotein obtained by incorporation of aldehyde, or lipoprotein which reacts with the antibody yielded by a hybridoma cell line FOH1a/DLH3 (Deposit No.: FERM BP-7171) (occasionally referred to simply as "DLH3 antibody"). The hybridoma cell line FOH1a/DLH3 which is used in the method described above was deposited on February 17, 1994 with Deposit No. FERM P-14153 in National Institute of Bioscience and Human-Technology, Agency of Industrial Science and Technology of the Ministry of International Trade and Industry located at 1-1-3, Higashi, Tsukuba-shi, Ibaraki-ken, Japan, under the designation of "Mouse-Mouse hybridoma FOH1a/DLH3," which deposit was switched on May 26, 2000 to the deposit based on the Budapest Treaty and has been stored at the institute with Deposit No. FERM BP-7171.

The conditions for the process including the step for freezing which is performed on the solution containing lipoprotein with a view to denaturing the lipoprotein in the present invention do not need to be particularly restricted but are only required to be capable of denaturing the lipoprotein contained in the solution. Further, the process including the step for freezing cannot be generally restricted because the effect of freezing is greatly swayed by the environment substantially encircling the lipoprotein during the course of the freezing. The conditions for the freezing in the process including the step for freezing, for example, are expected to permit a procedure which comprises lowering the temperature at a temperature decreasing speed in the range of 0.01°C - 10°C/min, preferably in the range of 0.01 °C - 1°C/min until it reaches a level in the range of 0°C - -196°C, preferably in the range of -5 °C - -85°C and freezes and then retaining a prescribed temperature for a period in the range of 0 - 36 hours, preferably in the range of 0 - 16 hours. In one embodiment, the process including the step for freezing for the purpose of denaturing lipoprotein is required to be performed at least once and, when necessary, may be repeated. When the process including the step for freezing is repeated, the number of repetitions is properly in the range of 1 - 10, preferably in the range of 1 - 4. In the present invention, the contents of the repeating processes may be the same or different at each step for freezing. The conditions for the step for freezing in each of the processes may be the same or different. If the number of repetitions of the step for freeze-drying exceeds 10, the excess will be at an economic disadvantage in inducing attenuation of the effect of denaturation of the lipoprotein.

The process including the step for freezing which aims to denature the lipoprotein in the first aspect of the invention described above incorporates therein a step for melting after the step for freezing. The conditions for the freezing do not need to be particularly restricted. The melting is attained by elevating the temperature at a temperature increasing speed in the range of 0.01°C - 10°C/min., preferably in the range of 0.1 °C - 10°C/min.until it reaches a level in the range of 0 °C - 42°C, preferably in the range of 0 °C - 37°C.

The process including the step for freezing for the purpose of denaturing lipoprotein according to the first aspect of the invention described above may be such a kind of process including a step for drying in the course of the process. The conditions for the process including the step for drying after the step for freezing do not need to be particularly restricted. The drying is carried out, for example, at a temperature in the range of -80 °C - 20°C, preferably in the range of -80 °C - 15°C, under a pressure in the range of 0.6 - 13 Pa, preferably in the range of 0.6 - 1.3 Pa, for a period in the range of 12 - 72 hours, preferably in the range of 24 - 72 hours.

A process including the step for freezing for the purpose of denaturing lipoprotein, may be such a kind of process as comprises freeze-drying a solution containing the lipoprotein, dissolving the resultant dried solution in a solvent, and subsequently again freeze-drying the produced solution. Such a process is not included within the scope of the present invention. The step for freeze-drying involved in this case has the same definition as defined above. The solvent for dissolving the product obtained by the first freeze-drying does not need to be particularly restricted but is only required to be capable of dissolving the dried product. As concrete examples of the solvent, water, deionized water, and distilled water may be cited.

The method according to the first aspect of the present invention may further comprise freeze drying the denatured lipoprotein obtained by the method of the first aspect of the present invention described above and consequently stabilizing the denatured lipoprotein. The step for freeze-drying according to the aspect described above has the same definition as in the first aspect with the exception of the timing of the addition of the stabilizing agent. To be more specific, the stabilizing agent varies in kind with the contents of the process including the step for freezing in the first aspect. When the process including the step for freezing is performed on the solution containing denatured lipoprotein, for example, the addition of the stabilizing agent may precede the process including the step for freezing or intervene between the step for freezing which is aimed at denaturing lipoprotein and the step for freeze-drying which is aimed at stabilizing the denatured lipoprotein. When the solution containing the denatured lipoprotein is to be frozen, the stabilizing agent is preferred to be added in advance of the process including the step for freezing.

The second aspect of the present invention consists in providing stabilized denatured lipoprotein that is produced by the first aspect of the invention described above.

The denatured lipoprotein and the stabilized denatured lipoprotein which are so produced excel in stability of prolonged preservation and, therefore, prove useful as a standard substance used in a method for determining denatured lipoprotein contained in a blood component by causing a given sample to contact an antibody capable of recognizing denatured lipoprotein and measuring the reactivity of the antibody with the sample or as a varying experimental reagent for investigating the physiological role or physiological activity of denatured lipoprotein.

The third aspect of the present invention, therefore, provides a method for the determination of denatured lipoprotein in a sample, comprising the steps of: selecting a stabilized denatured lipoprotein according to the second aspect as a standard substance; forming a calibration curve using prescribed concentrations of the standard substance; reacting the sample with an antibody which binds to the denatured lipoprotein; and measuring the reactivity of the antibody with the sample. The fourth aspect of the present invention comprises providing a reagent kit for the determination of denatured lipoprotein comprising stabilized denatured lipoprotein in accordance with the second aspect described above as a standard substance.

The method for determining the denatured lipoprotein in the third aspect described above may be selected from among the methods which have been heretofore known as useful for the purpose. As concrete examples of the method, such methods as radioimmunoassay (RIA), enzyme immunoassay (ELISA), fluoroimmunoassay (FIA), luminescent immunoassay, agglutination immunoassay, immunonephelometry, and nephelometric immunoassay which are immunologic determination of denatured lipoprotein contained in a blood component by causing a given sample to contact the lipoprotein to an antibody capable of recognizing the denatured lipoprotein and measuring the reactivity of the antibody with the sample may be cited. As the measuring method, competitive assay and sandwich technique may be cited. Among the methods enumerated above, such methods as radioimmuno-assay, enzyme immunoassay, fluorescent immunoassay, and luminescent immunoassay which effect the determination immunologically permit particularly favorable use of the stabilized denatured lipoprotein according to the present invention as a standard substance.

In the fourth aspect of the invention mentioned above, the reagent kit for the determination of denatured lipoprotein does not need to be particularly restricted but is only required to contain stabilized denatured lipoprotein according to the present invention as a standard substance. This reagent kit has the same structure as the known kit, except it uses stabilized denatured lipoprotein according to the present invention as a standard substance. When the stabilized denatured lipoprotein contemplated by the present invention is used as a standard substance in the ELISA method, for example, this reagent kit comprises a diluting liquid for a sample, a solid phase formed by immobilizing an antibody, a reaction buffer, a washing solution, a labeled secondary antibody (preferably an enzyme labeled secondary antibody), a detecting reagent (for example, a colouring fluid), and the whole or part of the stabilized denatured lipoprotein according to the present invention as a standard substance. The mode of embodiment just described is also embraced in the concept of the present invention. The fourth aspect of the present invention, therefore, also provides a reagent kit for the determination of denatured lipoprotein which comprises a diluting liquid for a sample, a solid phase formed by immobilizing an antibody, a reaction buffer, a washing solution, a labeled secondary antibody, a detecting reagent, and the whole or part of stabilized denatured lipoprotein according to the second aspect produced as a standard substance as component elements.

In the fourth aspect of this invention described above, when the standard substance is not contained in the reagent kit but is still retained on the precondition that it will be substantially used in a kit, the standard substance according to the present invention ought to be recognized as a component element for the reagent kit.

Now, the method for the production of denatured lipoprotein according to the present invention and the effect thereof will be described more specifically below with reference to the following working examples resorting to the ELISA method for the determination of the activity of denatured lipoprotein. Of course, the present invention does not need to be limited to the following working examples.

### Comparative Example 1

### (1) Preparation of LDL

From the human blood plasma obtained by using EDTA as an anticoagulant, a part having specific gravity in the range of 1.019 - 1.063 was collected as an LDL fraction by the ultracentrifugation technique (by adjusting a given sample to a specific gravity of 1.019 at 10°C, centrifuging the sample with 120,000xg for 20 hours, collecting the supernatant formed consequently, adjusting the supernatant to a specific gravity of 1.063, and further centrifuging it with 120,000xg for 24 hours). In this case, the purity of the LDL was confirmed by the fact that the sample formed a single sharp band when it was assayed by the agarose electrophoresis technique.

Then, this LDL fraction was purified by dialyzing thoroughly for 16 hours or overnight against PBS (pH 7.4) containing 0.25 mmol/L of EDTA. The LDL purified as described above was dissolved in PBS (pH 7.4) containing 0.25 mmol/L of EDTA until an LDL protein concentration reached 1 mg/mL and preserved in the dissolved state at 4°C until it was put to use. In the present example, the mass of protein was determined by the Lowry modified Method. To be precise, this determination was effected by preparing a reagent by mixing a solution containing 2 (w/v) %of sodium carbonate, 0.4 (w/v) % of sodium hydroxide, 0.16 (w/v) % of tartaric acid, 1 (w/v) % of SDS and a solution containing 4 (w/v) % copper sulfate at a ratio of 100 : 1, mixing this reagent of 1.5 mL respectively with a given samples or a standard substance (BSA) of 0.5 mL in volume, allowing the relevant components to react at room temperature for 20 minutes, immediately mixing the resultant reaction solution with 0.15 mL of phenol reagent, allowing them to react at room temperature for 45 minutes, and measuring the produced reaction for absorbance at 660 nm.

### (2) Oxidation of LDL

The LDL prepared in (1) was deprived of EDTA by dialyzing not less than 3 times (for not less than two hours/each) against not less than 500-1000 times as large in volume of PBS (pH 7.4) containing no EDTA and then dissolved in PBS (pH 7.4) containing no EDTA until the concentration of LDL reached 200 µg/mL. Then, 10 mL of the resultant LDL solution and copper sulfate (CuSO₄) added thereto until the final concentration reached 5 µmols/L were left incubating at 37°C for three hours to effect oxidation of the LDL. To add EDTA to the solution until the final concentration of EDTA reached 1 mmol/L, the oxidation was stopped. The solution was deprived of CuSO₄ by dialyzing not less than three times (for not less than two hours/each) against not less than 500-1000 times as large in volume of PBS (pH 7.4) containing 1 mmol/L of EDTA. The oxidized LDL consequently prepared was preserved at 4°C.

In the present working example, the expression of the amount of the oxidized LDL was defined by the mass of protein of the LDL as the raw material.

### (3) Preparation of freeze-dried product of oxidized LDL

The oxidized LDL prepared in (2) was diluted with PBS (pH 7.4) until the concentration of protein reached 6.25 ng/mL in one portion (referred to as "L type") and 12.5 ng/mL in another portion (referred to as "H type"). The two types were each mixed well with BSA added thereto until the final concentration reached 2 (w/v) % and sucrose was added thereto until the final concentration reached 5 (w/v) % respectively. Then, the produced mixed solutions were each dispensed in a unit volume of 1 mL into glass vials, frozen by the use of a vacuum freeze-drying device, Kyowa Drier RL-201BS (Kyowa Shinku Gijutsu K.K.) at -50°C for 16 hours, freeze-dried at 20°C under a reduced pressure of 1.33 Pa for 48 hours to gasify and expel the water component, then stoppered, and preserved at 4°C. At this time, each of the water contents of the freeze-dried products was 0.8 mass %.

### (4) Formation of calibration curve with the freeze-dried product of oxidized LDL as standard substance

### a. Production of peroxidase labeled antibody

One (1) mL of purified anti-human apo-B antibody (goat, Nippon Chemi-con Corp.) solution (5 mg/mL in concentration in 0.1 mol/L of borate buffer, pH 8.0) and 50 µL of 2-iminothiolan-HCl solution (60 mmol/L in concentration in 0.1 mol/L of borate buffer, pH 8.0) added thereto were left reacting with each other at 30°C for 30 minutes. The resultant reaction solution was treated with a sephadex G-25 column (1 cm × 30 cm) (Pharmarcia Corp.) equilibrated with 0.1 mol/L of phosphate buffer (pH 6.0) containing 5 mmol/L of EDTA. The antibody fraction eluted from the column was collected. One (1) mL of horseradish peroxidase (hereinafter abbreviated as "HRP," Toyobo K.K.) Solution (10 mg/mL in concentration in 0.1 mol/L of phosphate buffer, pH 7.0) and 50 µl of EMCS solution (50 mmol/L of DMSO solution, Dojin Kagakusha K.K.) added thereto were left reacting with each other at 30°C for 30 minutes. The resultant reaction solution was treated with a sephadex G-25 column (1 cm × 30 cm) (Pharmarcia Corp.) equilibrated with 0.1 mol/L of phosphate buffer (pH 6.5). The HRP fraction eluted from the column was collected. The antibody fraction and the HRP fraction collected as described above were mixed and left reacting with each other at 30°C for 30 minutes. The resultant reaction solution was treated with a sephadex G-200 column (1 cm × 100 cm) (Pharmarcia Corp.) equilibrated with 0.1 mol/L of phosphate buffer (pH 7.0). The antibody-HRP conjugate fractions eluted from the column were mixed and collected. The mass consequently collected was designated as "peroxidase-labelled anti-human apo-B antibody." The collected fraction was immediately diluted with BSA until a final concentration reached 1 (w/v) % and preserved in the diluted state at -50°C until use.

### b. Preparation of DLH3 antibody

Male Balb/c mice not less than 8 weeks old, after administration by intra-abdominal injection of pristane (2,6,10,14-tetramethylpentadecane) with a dose of 0.5 mL/individual were each reared for two weeks. Then, the mice were each administrated to intra-abdominally with a hybridoma cell line FOH1a/DLH3, a cell capable of yielding a required monoclonal antibody (Deposit No. FERM BP-7171; J.Biol. Chem. 1994, 269: 15274-15279; and Patent publication JP-A-07-238,098) with a dose of 1 × 10⁶/individual. 7-14 days later, when the mice each accumulated ascites amply in the abdomen, the ascites was collected from the abdomen by the use of an 18G syringe and centrifuged at 3000 rpm for 10 minutes. The supernatant consequently formed was collected. To this supernatant, an equal amount of PBS (pH 7.4) was added. To the produced mixture, a saturated ammonium sulfate solution equal in amount to the mixture was added dropwise over a period of one hour as kept thoroughly stirred. The resultant mixture was continuously stirred further for one hour and then centrifuged at 3000 rpm for 10 minutes to discard the supernatant and collect the sediment. Further, the sediment was dissolved in PBS (pH 7.4) containing 0.5 mol/L of NaCl. The produced solution was treated with a Sephacryl S-300 column (2.5 cm × 100 cm) (Pharmacia Corp.) equilibrated with PBS (pH 7.4) containing 0.5 mol/L of NaCl to collect a IgM fraction, which was designated as "DLH3 antibody." The concentration of the DLH3 antibody (mg/mL) was determined by measuring a given sample for absorbance at 280 nm in a light path 1 cm in length and dividing the obtained absorbance by 1.3.

### c. Sandwich ELISA analysis

The freeze-dried product of the oxidized LDL prepared in (3) was dissolved in 1 mL of purified water and then diluted with PBS containing 1 (w/v) % of BSA till varying stated concentrations (0 ng/mL, 3.125 ng/mL, 6.25 ng/mL, 12.5 ng/mL, and 25 ng/mL).

In the individual wells of a 96F microplate (NALGE NUNC Inaternational K.K.), the DLH3 antibody prepared in b. above and diluted with Tris-HCl (pH 8.0) to 10 µg/mL was dispensed in a unit amount of 1 µg/well and incubated at 4°C for 16 hours. The antibody solution formed in the wells was discarded. The residue in each of the wells was blocked by being incubated together with 350 µL of Tris-HCl (pH 8.0) containing 1 (w/v) % of BSA at room temperature for two hours. The blocked antibody solution was washed four timed with PBS (pH 7.4) containing 0.05 (v/v) % of Tween20.

The diluted solution containing the freeze-dried product of oxidized LDL of a prescribed concentration prepared as described above was dispensed in a unit volume of 100 µL to the individual wells, incubated at room temperature for two hours, and then washed four times with PBS (pH 7.4) containing 0.05 (v/v) % of Tween20.

To the individual wells, 100 µL of the solution obtained by diluting the peroxidase labeled anti-human apo-B antibody prepared in "a." above with PBS (pH 7.4) containing 1 (w/v) % of BSA to a volume of 1000 times the original volume was placed and incubated at room temperature for 30 minutes. The incubated solution was washed four times with PBS (pH 7.4) containing 0.05 (v/v) % of Tween20 and then left tinting for 30 minutes with 100 µL of 0.03 (w/v) % of an aqueous hydrogen peroxide solution containing 3 mg/mL of o-phenylenediamine (Wako Pure Chemical Industries Ltd.). The reaction still continuing in the solution was stopped by the addition of 50 µL of 1 mol/L sulfuric acid. The solution was then measured absorbance at 492 nm. The results are shown in Fig. 1. By the use of the freeze-dried product of oxide LDL of the present invention, a fine calibration curve could be formed as illustrated in Fig. 1.

### (5) Comparison of stability of preservation

The freeze-dried products of the oxidized LDL prepared in (3) [standard oxidized LDL (1) (H type), 12.5 ng and standard oxidized LDL (2) (L type), 6.25 ng] were preserved at 4°C for stated periods (0, 1, 3, and 4 weeks). The oxidized LDL prepared in (2) was diluted with PBS (pH 7.4) to two protein concentrations of 6.25 ng/mL (hereinafter referred to as "L type") and 12.5 ng/mL (hereinafter referred to as "H type"). BSA and sucrose were added to the two types to the final concentration of 2(w/v) % and 5 (w/v) % respectively, and the resultant solutions were mixed well. The resultant mixed solutions were preserved in cold storage at 4°C without being freeze-dried [i.e. the oxidized LDL for comparison (1) (H type), 12.5 ng/mL and the oxidized LDL for comparison (2) (L type), 6.25 ng/mL] were preserved at 4°C for stated periods (0, 1, 3, and 4 weeks).

After the preservation for the stated period, the standard oxidized LDLs (1) and (2) each dissolved in 1 mL of purified water and the oxidized LDLs for comparison (1) and (2) were treated by the same procedure as in (4) c. above and measured absorbance at 492 nm. The results are shown in Fig. 2.

As shown in Fig. 2, the measurements obtained from the standard oxidized LDLs (1) and (2) showed practically no change from those obtained immediately after preparation (0 week), whereas the measurements obtained of the oxidized LDLs for comparison (1) and (2) after four weeks' preservation respectively showed falls of about 50% and about 45% from the measurements obtained immediately after preparation (0 week). These results clearly indicate that these oxidized LDLs for comparison were quite inferior in stability of preservation to the standard oxidized LDLs (1) and (2).

### Comparative Example 2

### (1) Preparation of HDL

From the human blood plasma obtained by using EDTA as an anticoagulant, LDL was removed and a part having specific gravity in the range of 1.063 - 1.21 was collected as an HDL fraction by the ultracentrifugation technique (with 120, 000×g at 10°C for 48 hours). In this case, the purity of the HDL was confirmed by the fact that the sample formed a single sharp band when it was assayed by the agarose electrophoresis technique.

Then, this HDL fraction was purified by dialyzing thoroughly for 16 hours against PBS (pH 7.4) containing 0.25 mmol/L of EDTA. The HDL purified as described above was dissolved in PBS (pH 7.4) containing 0.25 mmol/L of EDTA until an HDL protein concentration reached 1 mg/mL and preserved in the dissolved state at 4°C until it was put to use. In the present example, the mass of protein was determined by the Lowry modified Method described in comparative Example 1

### (2) Oxidation of HDL

The HDL prepared in (1) was deprived of EDTA by dialyzing not less than 3 times (for not less than two hours/each) against not less than 500-1000 times as large in volume of PBS (pH 7.4) containing no EDTA and then dissolved in PBS (pH 7.4) containing no EDTA until the concentration of HDL reached 100 µg/mL. Then, 10 mL of the resultant HDL solution and copper sulfate (CuSO₄) added thereto until the final concentration reached 10 µmol/L were left incubating at 37°C for 18 hours to effect oxidation of the HDL. To add EDTA to the solution until the final concentration of EDTA reached 1 mmol/L, the oxidation was stopped. The solution was deprived of CuSO₄ by dialyzing not less than three times (for not less than two hours/each) against not less than 500-1000 times as large in volume of PBS (pH 7.4) containing 1 mmol/L of EDTA. The oxidized HDL consequently prepared was preserved at 4°C.

In the present working example, the expression of the amount of the oxidized HDL was defined by the mass of protein of the HDL as the raw material.

### (3) Preparation of freeze-dried product of oxidized HDL

BSA and sucrose were added to the oxidized HDL prepared in (2) until the final concentration reaches 2 (w/v) % and 5(w/v) % respectively, and the resultant solution was mixed well. Then, the produced mixed solution was dispensed in a unit volume of 1 mL into glass vials, frozen by the use of a vacuum freeze-drying device, Kyowa Drier RL-201BS (Kyowa Shinku Gijutsu K.K.) at -50°C for 16 hours, freeze-dried at 20°C under a reduced pressure of 1.33 Pa for 48 hours to gasify and expel the water component, then stoppered, and preserved at 4°C. At this time, each of the water contents of the freeze-dried products was 0.8 mass %.

### (4) Formation of calibration curve with the freeze-dried product of oxidized HDL as standard substance

### a. Adjustment of concentration

The freeze-dried product of the oxidized HCL prepared in (3) was dissolved in 1 mL of purified water added thereto and diluted with PBS containing 1 (w/v) % of BSA to varying stated concentrations (0 ng/mL, 3.125 ng/Ml, 6.25 ng/mL, 12.5 ng/mL, 25 nm/mL, 50 ng/mL, and 75 ng/mL).

### b. Sandwich ELISA analysis

In the individual wells of a 96F microplate (NALGE NUNC Inaternational K.K.), an anti-human apo-AI mouse monoclonal antibody (Nippon Chemi-con Corp.) solution diluted with a carbonate buffer (pH 9.5) to a concentration of 10 µg/mL was dispensed in a unit volume of 0.1 mL/well and incubated at 4°C for 16 hours. The antibody solution formed in the wells was discarded. The residue in each of the wells was blocked by being incubated together with 350 µL of PBS (pH 7.4) containing 1 (w/v) % of BSA at room temperature for two hours. The blocked antibody solution was washed four times with PBS (pH 7.4) containing 0.05 (v/v) % of Tween20.

The diluted solution containing the freeze-dried product of oxidized HDL of a prescribed concentration prepared as described in "a." above was dispensed in a unit volume of 100 µL to the individual wells, incubated at room temperature for two hours, and then washed four times with PBS (pH 7.4) containing 0.05 (v/v) % of Tween20.

To the individual wells, a DLH3 antibody diluted with PBS (pH 7.4) containing 1 (w/v) % of BSA was dispensed in a unit volume of 100 µL and incubated at room temperature for one hour. The incubated solution was washed four times with PBS (pH 7.4) containing 0.05 (v/v) % of Tween20 and then incubated together with 100 µL of a peroxidase labeled anti-mouse IgM antibody (Zymed Laboratories, Inc.) diluted to 1000 times with PBS containing 1 (w/v) % BSA at room temperature for 30 minutes. The resultant incubated solution was washed four times with PBS (pH 7.4) containing 0.05 (v/v) % of Tween20 and then left tinting with 100 µL of 0.03 (w/v) % of an aqueous hydrogen peroxide solution containing 3 mg/mL of o-phenylenediamine. The reaction still continuing in the solution was stopped by the additionof 50 µL of 1 mol/L sulfuric acid. The solution was then measured absorbance at 492 nm. The results are shown in Fig. 3. By the use of the freeze-dried product of oxide HDL of the present invention, a fine calibration curve could be formed as illustrated in Fig. 3.

### (5) Comparison of stability of preservation

The freeze-dried products of the oxidized HDL prepared in (3) [standard oxidized HDL (1) (H type), 12.5 ng and standard oxidized HDL (2) (L type), 6.25 ng] were preserved at 4°C for stated periods (0, 1, 2, 3, and 4 weeks). The oxidized HDL prepared in (2) was diluted with PBS (pH 7.4) to two protein concentrations of 6.25 ng/mL (hereinafter referred to as "L type") and 12.5 ng/mL (hereinafter referred to as H type"). BSA and sucrose were added to the two types to the final concentration of 2(w/v)% and 5(w/v)% respectively, and the resultant solutions were mixed well. The resultant mixed solutions were preserved in cold storage at 4°C without being freeze-dried [i.e. the oxidized HDL for comparison (1) (H type), 12.5 ng/mL and the oxidized HDL for comparison (2) (L type), 6.25 ng/mL] were preserved at 4°C for stated periods (0, 1, 2, 3, and 4 weeks).

After the preservation for the stated period, the standard oxidized HDLs (1) and (2) each dissolved in 1 mL of purified water and the oxidized HDLs for comparison (1) and (2) were treated by the same procedure as in (4) b. above and measured absorbance at 492 nm. The results are shown in Fig. 4.

As shown in Fig. 4, the measurements obtained from the standard oxidized HDLs (1) and (2) showed practically no change from those obtained immediately after preparation (0 week), whereas the measurements obtained of the oxidized HDLs for comparison (1) and (2) after cold storage respectively showed falls of about 40% and about 30% from the measurements obtained immediately after preparation (0 week). These results clearly indicate that these oxidized HDLs for comparison were quite inferior in stability of preservation to the standard oxidized HDLs (1) and (2).

### Comparative Example 3

### (1) Preparation of Lp(a)

From the human blood plasma obtained by using EDTA as an anticoagulant, a part having specific gravity in the range of 1.060 - 1.125 was collected and further subjected to gel filtration with Biogel A-5m (Bio-rad Corp.) to collect a Lp(a) fraction. In this case, the purity of the Lp(a) was confirmed by the fact that the sample formed a single sharp band when it was assayed by the agarose electrophoresis technique.

Then, this Lp(a) fraction was purified by dialyzing thoroughly (for 16 hours) against PBS (pH 7.4) containing 0.25 mmol/L of EDTA. Further, the Lp(a) purified as described above was dissolved in PBS (pH 7.4) containing 0.25 mmol/L of EDTA until an Lp(a) protein concentration reached 1 mg/mL and preserved in the dissolved state at 4°C until it was put to use. In the present example, the mass of protein was determined by the Lowry modified Method.

### (2) Oxidation of Lp(a)

TheLp(a) prepared in (1) was deprived of EDTA by dialyzing not less than 3 times (for not less than two hours/each) against not less than 500-1000 times as large in volume of PBS (pH 7.4) containing no EDTA and then dissolved in PBS (pH 7.4) containing no EDTA until the concentration of Lp(a) reached 100µg/mL. Then, 10 mL of the resultant LDL solution and copper sulfate (CuSO₄) added thereto until the final concentration reached 10µmols/L were together left incubating at 37°C for 18 hours to effect oxidation of the Lp(a). To add EDTA to the solution until the final concentration of EDTA reached 1 mmol/L, the oxidation was stopped. The solution was deprived of CuSO₄ by dialyzing 2-3 times (for not less than two hours/each) against not less than 500-1000 times as large in volume of PBS (pH 7.4) containing 1 mmol/L of EDTA. The oxidized Lp(a) consequently prepared was preserved at 4°C.

In the present working example, the expression of the amount of the oxidized Lp(a) was defined by the mass of protein of the Lp(a) as the raw material.

### (3) Preparation of freeze-dried product of oxidized Lp(a)

BSA and sucrose were added to the oxidized Lp(a) prepared in (2) until the final concentration reaches 2 (w/v) % and 5(w/v) % respectively, and the resultant solution was mixed well. The resultant mixture was dispensed in a unit volume of 1 mL into glass vials, then frozen at -50°C for 16 hours by the use of a vacuum freeze-drying device, Kyowa Drier RL-201BS (Kyowa Shinku Gijutsu K.K.), freeze-dried at 20°C under a reduced pressure of 1.33 Pa for 48 hours to gasify and expel the water component, then stoppered, and preserved at 4°C. At this time, each of the water contents of the freeze-dried products was 0.8 mass %.

### (4) Formation of calibration curve with the freeze-dried product of oxidized Lp(a) as standard substance

### a. Adjustment of concentration

The freeze-dried product of the oxidized Lp(a) prepared in (3) was dissolved in 1 mL of purified water to varying stated concentrations (0 ng/mL, 0.15625 ng/mL, 0.3125 ng/mL, 0.625 ng/mL, 1.25 ng/mL, 2.5 ng/mL, and 5 ng/mL).

### b. Sandwich ELISA analysis

In the individual wells of a 96F microplate (NALGE NUNC Inaternational K.K.), an anti-human Lp(a) mouse monoclonal antibody (Nippon Chemi-con Corp.) solution diluted with a carbonate buffer (pH 9.5) to a concentration of 10 µg/mL was dispensed in a unit amount of 1µg/well and incubated at 4°C for 16 hours. The antibody solution formed in the wells was discarded. The residue in each of the wells was blocked by being incubated together with 350 µL of PBS (pH 7.4) containing 1 (w/v) % of BSA at room temperature for two hours. The blocked antibody solution was washed four timed with PBS (pH 7.4) containing 0.05 (v/v) % of Tween20.

The aqueous solution containing the freeze-dried product of the oxidized Lp(a) of a prescribed concentration prepared as described in "a." above was dispensed in a unit volume of 100 µL to the individual wells, incubated at room temperature for two hours, and then washed four times with PBS (pH 7.4) containing 0.05 (v/v) % of Tween20.

In the individual wells, the diluted solution obtained by diluting the DLH3 antibody prepared in (4) b. of comparative Example 1 to a concentration of 10 µg/mL with a 20 mmol/L of Tris-HCl solution (pH 7.4) containing 1 (w/v) % of BSA was dispensed in a unit volume of 100 µL and incubated at room temperature for one hour. Then, the incubated solution was washed four times with PBS (pH 7.4) containing 0.05 (v/v) % of Tween20 and then incubated together with 100 µL of a peroxidase labeled anti-mouse IgM antibody (Nippon Chemi-con Corp.) diluted to 1000 times with PBS containing 1 (w/v) % BSA at room temperature for 30 minutes. The resultant incubated solution was washed four times with PBS (pH 7.4) containing 0.05 (v/v) % of Tween20 and then left tinting with 100 µL of 0.03 (w/v) % of an aqueous hydrogen peroxide solution containing 3 mg/mL of o-phenylenediamine. The reaction still continuing in the solution was stopped by the addition of 50 µL of 1 mol/L sulfuric acid. The solution was then measured absorbance at 492 nm. The results are shown in Fig. 5. By the use of the freeze-dried product of oxide Lp(a) of the present invention, a fine calibration curve could be formed as illustrated in Fig. 5.

### (5) Comparison of stability of preservation

The freeze-dried products of the oxidized Lp(a) prepared in (3) [standard oxidized Lp(a) (1) (H type), 12.5 ng and standard oxidized Lp(a) (2) (L type), 6.25 ng] were preserved at 4°C for stated periods (0, 1, 2, 3, and 4 weeks). The oxidized Lp(a) prepared in (2) was diluted with PBS (pH 7.4) to two protein concentrations of 6.25 ng/mL (hereinafter referred to as "L type") and 12.5 ng/mL (hereinafter referred to as "H type"). BSA and sucrose were added to the two types to the final concentration of 2(w/v)% and 5(w/v)% respectively, and the resultant solutions were mixed well. The resultant mixed solutions were preserved in cold storage at 4°C without being freeze-dried [i.e. the oxidized Lp(a)s for comparison (1) (H type), 12.5 ng/mL and the oxidized Lp(a) for comparison (2) (L type), 6.25 ng/mL] were preserved at 4°C for stated periods (0, 1, 2, 3, and 4 weeks).

After the preservation for the stated period, the standard oxidized Lp(a)s (1) and (2) each dissolved in 1 mL of purified water and the oxidized Lp(a)s for comparison (1) and (2) were treated by the same procedure as in "(4) c." above and measured absorbance at 492 nm. The results are shown in Fig. 6.

As shown in Fig. 6, the measurements obtained from the standard oxidized Lp(a)s (1) and (2) showed practically no change from those obtained immediately after preparation (0 week), whereas the measurements obtained of the oxidized Lp (a)s for comparison (1) and (2) after four weeks' cold storage respectively showed falls of about 50% and about 40% from the measurements obtained immediately after preparation (0 week). These results clearly indicate that these oxidized Lp(a)s for comparison were quite inferior in stability of preservation to the standard oxidized Lp(a)s (1) and (2).

### Example 1

### (1) Preparation of freeze-denatured human blood plasma

Blood samples were drawn from four subjects with heparin as an anticoagulant. Blood plasma samples were collected respectively from the blood samples by the ordinary method and assayed by a method similar to the ELISA method described in comparative Example 1 (4).

Then, the blood plasma samples were each dispensed in a unit volume of 2 mL into glass vials, which were transferred from room temperature (25°C) to a freezer having an inner temperature of -30°C, left standing therein for not less than three hours, returned to room temperature, and completely melted the whole contents. The individual blood plasma that had undergone the process including the freezing operation was analyzed by a procedure similar to the ELISA method described in comparative Example 1 (4). The results are shown in Fig. 7. Fig. 7 clearly indicates that when the process including the freezing operation was performed on the blood plasma, the lipoprotein contained in the blood plasma was significantly denatured to produce a freeze-denatured lipoprotein (oxidized LDL).

### (2) Preparation of freeze-dried product of freeze-denatured human blood plasma

The blood plasma samples that had undergone the process including total 4 times of the freezing operation described in (1) above were mixed in respectively equal volumes. The denatured LDL (oxidized LDL) concentration of the resultant mixed solution was calculated from the calibration curve formed as illustrated in comparative Example 1 (4) in accordance with the ELISA method described in comparative Example 1 (4).

Then, the freeze-denatured human blood plasma prepared in (1) was diluted to two oxidized LDL concentrations, 6.25 ng/mL (L type) and 12.5 ng/mL (H type), with a 10 mmol/L phosphate buffer (pH 7.4) containing 140 mmol/L of NaCl, BSA of final concentration of 2 (w/v) %, sucrose of final concentration of 5 (w/v) %, and 0.25 mmol/L of EDTA-2Na. The two types of diluted solution were dispensed in a unit volume of 1 mL into glass vials, frozen at-50°C for 16 hours by the use of a vacuum freeze-drying device, Kyowa Drier RL-201BS (Kyowa Shinku Gijutsu K.K.), then freeze-dried at 5°C under a reduced pressure of 1.33 Pa for 48 hours to gasify and expel the water component, stoppered, and preserved at 4°C until use. This was designated as "freeze-dried product of freeze-denatured human blood plasma." Each of the water contents of the freeze-dried products was 0.8 mass %.

### (3) Determination of concentration of freeze-dried product of freeze-denatured human blood plasma

The freeze-dried product of the freeze-denatured human blood plasma prepared in (2) was dissolved in 1 mL of purified water added thereto. The resultant solution was measured absorbance at 492 nm in accordance with the method for determining the oxidized LDL by the sandwich ELISA method described in comparative Example 1 (4). When the oxidized LDL concentration of the solution was sought from the calibration curve formed in comparative Example 1 (4) based on the absorbance found by the measurement, the oxidized LDL concentration in the freeze-dried product showed practically no discernible change before and after the step of freeze-drying.

### (4) Comparison of stability of preservation

The two types of freeze-dried product [standard freeze-denatured human blood plasma (1) (H type), 12.5 ng and standard freeze-denatured human blood plasma (2) (L type), 6.25 ng] of the freeze-denatured human blood plasma prepared in (2) were preserved at 4°C for varying stated periods (0, 6, and 12 months). Then, the two types of freeze-dried product of oxidized LDL [standard oxidized LDL (1) (H type), 12.5 ng and standard oxidized LDL (2) (L type), 6.25 ng] prepared in comparative Example 1 (3) were preserved at 4°C for varying stated periods (0, 6, and 12 months).

After the preservation for the stated period, the standard freeze-denatured human blood plasmas (1) and (2) and the standard oxidized LDLs (1) and (2) were each dissolved in 1 mL of purified water. The resultant solutions were assayed by following a procedure similar to the ELISA method described in comparative Example 1 (4). The results are shown in Table 1 below.

**Table 1**

| Duration of preservation (months) | OD₄₉₂ | | | |
|---|---|---|---|---|
| | Standard freeze-denatured human blood plasma (1) | Standard freeze-denatured human blood plasma (2) | Standard oxidized LDL (1) | Standard oxidized LDL (2) |
| 0 | 0.740 | 0.272 | 0.758 | 0.283 |
| 6 | 0.727 | 0.270 | 0.508 | 0.222 |
| 12 | 0.677 | 0.274 | 0.430 | 0.207 |

As shown in Table 1, the standard oxidized LDL excelled in stability of preservation so long as it had a low concentration [standard oxidized LDL (2)]. The standard freeze-denatured human blood plasma according to the present invention which attained denaturation of lipoprotein by freezing and then acquired stability by freeze-drying significantly excelled compared with the standard oxidized LDL in terms of stability of prolonged preservation even when it had a high concentration [standard freeze-denatured human blood plasma (1)] compared with the standard oxidized LDL.

### Comparative Example 4

### (1) Preparation of freeze-denatured human blood serum

Blood samples were drawn from four subjects. Blood serum samples were separated respectively from the blood samples by the ordinary method and mixed. BSA and sucrose were added to the blood serum to the final concentration of 2(w/v)% and 5(w/v)% respectively, and the resultant mixtures were mixed well. The mixtures were dispensed in a unit volume of 2 mL into glass vials, frozen by the use of a vacuum freeze-drying device, Kyowa Drier RL-201BS (Kyowa Shinku Gijutsu K.K.) at -50°C for 16 hours, freeze-dried at 5°C under a reduced pressure of 1.33 Pa for 48 hours to gasify and expel the water component, stoppered, and preserved at 4°C. Each of the water contents of the freeze-dried products was 0.8 mass %.

### (2) Preparation of freeze-dried product of freeze-denatured human blood serum

Purified water was added in a unit volume of 2 mL to the glass vials containing the freeze-denatured human blood plasma samples prepared in (1), thereby dissolved the contents of the vials thoroughly. The oxidized LDL concentrations of the produced solutions were calculated from the calibration curve formed as illustrated in comparative Example 1 (4) in accordance with the ELISA method described in comparative Example 1 (4).

Then, the freeze-denatured human blood serum prepared in (1) was diluted to two oxidized LDL concentrations, 6.25 ng/mL (L type) and 12.5 ng/mL (H type), with a 10 mmol/L phosphate buffer (pH 7.4) containing 140 mmol/L of NaCl, BSA of final concentration of 2 (w/v) %, sucrose of final concentration of 5 (w/v) %, and 0.25 mmol/L of EDTA-2Na. The two types of diluted solution were dispensed in a unit volume of 1 mL into glass vials, frozen at-50°C for 16 hours by the use of a vacuum freeze-drying device, Kyowa Drier RL-201BS (Kyowa Shinku Gijutsu K.K.), then freeze-dried at 5°C under a reduced pressure of 1.33 Pa for 48 hours to gasify and expel the water component, stoppered, and preserved at 4°C until use. This was designated as "freeze-dried product of freeze-denatured human blood plasma. " Each of the water contents of the freeze-dried products was 0.8 mass %.

### (3) Determination of concentration of freeze-dried product of freeze-denatured human blood serum

The freeze-dried product of the freeze-denatured human blood serum prepared in (2) was dissolved in 1 mL of purified water added thereto. When the oxidized LDL concentration of the resultant solution was sought from the calibration curve formed in comparative Example 1 (4) by the method for determination of the oxidized LDL in accordance with the ELISA method described in comparative Example 1 (4), the oxidized LDL concentration in the freeze-dried product showed practically no discernible change before and after the step of freeze-drying.

### (4) Comparison of stability of preservation

The freeze-dried product of the freeze-denatured human blood serum prepared in (2) was dissolved with 1 mL of purified water into two oxidized LDL concentrations of 12.5ng/mL and 6.25ng/mL respectively to prepare the standard freeze-denatured human blood serum samples, (1) (H type) and (2) (L type). These two types of samples were preserved at 4°C for varying stated periods (0, 3, 7, 10, and 14 days). Then, the two types of freeze-dried product of the oxidized LDL prepared in comparative Example 1 (3) were dissolved in the same manner as described above to obtain standard oxidized LDLs having oxidized LDL concentrations of 12.5 ng/mL and 6.25 ng/mL i.e., standard oxidized LDL (3) (H type) and standard oxidized LDL (4) (L type). These types were preserved in a dissolved state at 4°C for varying stated periods (0, 3, 7, 10, and 14 days).

After the preservation for the stated period, the standard freeze-denatured human blood plasmas (1) and (2) and the standard oxidized LDLs (3) and (4) were each assayed by following the method for determination of oxidized LDL in accordance with the sandwich ELISA method described in comparative Example 1 (4). The results are shown in Table 2 and Fig. 8.

**Table 2**

| Duration of preservation (days) | OD₄₉₂ | | | |
|---|---|---|---|---|
| | Standard freeze-denatured human blood serum (1) | Standard freeze-denatured human blood serum (2) | Standard oxidized LDL (3) | Standard oxidized LDL (4) |
| 0 | 0.8175 | 0.3693 | 0.7689 | 0.3608 |
| 3 | 0.8107 | 0.3533 | 0.6762 | 0.3615 |
| 7 | 0.7602 | 0.3387 | 0.6705 | 0.3473 |
| 10 | 0.7800 | 0.3440 | 0.5772 | 0.3240 |
| 14 | 0.7630 | 0.3400 | 0.5760 | 0.2985 |

As shown in Table 2, the standard oxidized LDL excelled in stability of preservation so long as it had a low concentration [standard oxidized LDL (4)]. The standard freeze-denatured human blood serum which attained denaturation of lipoprotein by freezing and then acquired stability by freeze-drying significantly excelled compared with the standard oxidized LDL in terms of stability of prolonged preservation even when it had a high concentration [standard freeze-denatured human blood serum (1)].

### Example 2

### (1)Preparation of freeze-denaturated LDL

The LDL prepared in comparative Example 1 (1) was deprived of EDTA by dialyzing not less than three times (not less than two hours/each) against not less than 500 - 1000 times as large in volume of PBS (pH 7.4) containing no EDTA, diluted to a LDL concentration of 1 mg/mL with a 10 mmol/L phosphate buffer (pH 7.4) containing 140 mmol/L of NaCl, BSA of a final concentration of 2 (w/v) %, sucrose of a final concentration of 5 (w/v) %, and 0.25 mmol/L of EDTA-2Na, and mixed well. Then, the produced diluted solution was dispensed in a unit volume of 2 mL into glass vials, which were transferred from room temperature (22°C) to freezers having inner temperatures of -85°C and -30°C respectively, left standing therein for not less than one hour, and returned to room temperature to effect thorough dissolution of the contents of the vials. The process including the step of freezing and the dissolving mentioned above was performed up to a total of 10 repetitions. The contents of the glass vials were partly collected during some intervals between the said processes including the steps of freezing and melting. The samples thus obtained were assayed by the sandwich ELISA method described in comparative Example 1 (4). The results are shown in Fig. 9.

As shown in Fig. 9, the denaturation of LDL nearly reached a saturated state when the process including the steps of freezing and melting was performed up to three repetitions while the internal temperature of the freezer was -30°C. When the internal temperature of the freezer was -85°C, however, the denaturation of LDL did not reach a saturated state unless the process including the steps of freezing and melting was performed up to nine repetitions. The results indicate that the product amount of denatured LDL to be produced varies with the lowered temperature.

### (2) Preparation of freeze-dried product of freeze-denatured LDL

The freeze-denatured LDL prepared by performing the process including the step of freezing at the internal temperature, -30°C, of the freezer in (1) up to a total of four repetitions was diluted to oxidized LDL concentrations, 6.25 ng/mL (L type) and 12.5 ng/mL (H type), with 10 mmol/L phosphate buffer (pH 7.4) containing 140 mmol/L of NaCl, BSA of final concentration of 2 (w/v) %, sucrose of final concentration of 5 (w/v) %, and 0.25 mmol/L of EDTA-2Na. The two types of diluted solution thus obtained were dispensed in a unit volume of 1 mL into glass vials, frozen at -50°C for 16 hours by the use of a vacuum freeze-drying device, Kyowa Drier RL-201BS (Kyowa Shinku Gijutsu K.K.), then freeze-dried at 5°C under a reduced pressure of 1.33 Pa for 48 hours to gasify and expel the water component, stoppered, and preserved at 4°C until use. This was designated as "freeze-dried product of freeze-denatured LDL. Each of the water contents of the freeze-dried products was 0.8 mass %.

### (3) Determination of concentration of freeze-dried product of freeze-denatured LDL

The freeze-dried product of the freeze-denatured LDL prepared in (2) was dissolved in 1 mL of purified water added thereto. When the oxidized LDL concentration of the resultant solution was sought from the calibration curve formed in comparative Example 1 (4) by the method for determination of the oxidized LDL in accordance with the ELISA method described in comparative Example 1 (4), the oxidized LDL concentration in the freeze-dried product showed practically no discernible change before and after the step of freeze-drying.

### (4) Comparison of stability of preservation after dissolution

The freeze-dried product of the freeze-denatured LDL prepared in (2) was dissolved with 1 mL of purified water into two oxidized LDL concentrations, 12.5 ng/mL and 6.25 ng/mL, to prepare the standard freeze-denatured human LDLs, (1) (H type) and (2) (L type) respectively. These two types of samples were preserved at 4°C for varying stated periods (0, 3, 7, 10, and 14 days). Then, the two types of freeze-dried product of the oxidized LDL prepared in comparative Example 1 (3) were dissolved in the same manner as described above to obtain standard oxidized LDLs having oxidized LDL concentrations of 12.5 ng/mL and 6.25 ng/mL [standard oxidized LDL (3) (H type) and standard oxidized LDL (4) (L type)]. These types were preserved in a dissolved state at 4°C for varying stated periods (0, 3, 7, 10, and 14 days)

After the preservation for the stated period, the standard freeze-denatured LDLs (1) and (2) and the standard oxidized LDLs (3) and (4) were each measured absorbance at 492 nm by following the method for determination of oxidized LDL in accordance with the sandwich ELISA method described in comparative Example 1 (4). The oxidized LDL contents in the standard samples were sought from the calibration curve formed in comparative Example 1 (4), based on the absorbance obtained by the measurement above. The results are shown in Table 3 and Fig. 10.

**Table 3**

| Duration of preservation (days) | OD₄₉₂ | | | |
|---|---|---|---|---|
| | Standard freeze-denatured LDL (1) | Standard freeze-denatured LDL (2) | Standard oxidized LDL (3) | Standard oxidized LDL (4) |
| 0 | 0.8633 | 0.3826 | 0.7689 | 0.3608 |
| 3 | 0.8265 | 0.3532 | 0.6762 | 0.3615 |
| 7 | 0.7924 | 0.3516 | 0.6705 | 0.3473 |
| 10 | 0.7952 | 0.3439 | 0.5772 | 0.3240 |
| 14 | 0.7886 | 0.3476 | 0.5760 | 0.2985 |

As shown in Table 3, the standard oxidized LDL invention excelled in stability of preservation so long as it had a low concentration [standard oxidized LDL (4)]. The standard freeze-denatured human blood serum according to the present invention which attained denaturation of lipoprotein by freezing and then acquired stability by freeze-drying significantly excelled compared with the standard oxidized LDL in terms of stability of prolonged preservation after dissolution even when it had a high concentration [standard freeze-denatured LDL (1)].

### Industrial Applicability of the Invention

It is strongly suggested that the denatured lipoprotein is deeply related to various diseases of the circulatory system including such diseases of the coronary artery system as cardiac infarction and stenocardia, such diseases of the cerebral arteries as cerebral infarction and cerebrovascular dementia, such diseases of the renal arteries as nephropathy and diabetic nephropathy, and such diseases of the peripheral artery system as obstruction of peripheral arteries. The standard substance for the determination of denatured lipoprotein in blood and the reagent for the investigation of the physiological role and the physiological activity of denatured lipoprotein are very important substances which affect the results of such experiments.

By the method of the present invention, therefore, it has now become possible to produce denatured lipoprotein excelling in stability of preservation, to put it in other words, exhibiting definite determinations. In addition to the advantage mentioned above, the stabilized lipoprotein which is produced by freeze-drying denatured lipoprotein obtained by performing a method of the first aspect excels not only in stability of preservation but also in stability of preservation after dissolution. The stabilized denatured lipoprotein contemplated by the present invention retains the excellent stability even when it is applied to the form of a solution, i.e. the forms as actual use. This fact makes this stabilized denatured lipoprotein highly advantageous for the sake of determining denatured lipoprotein.

The stabilized denatured lipoprotein according to the present invention, therefore, is useful as a standard substance in a method for determining denatured lipoprotein contained in a blood component by placing a given sample in contact with an antibody capable of recognizing denatured lipoprotein and measuring the reactivity of this antibody on the sample and as a varying experimental reagent for investigating the physiological role and the physiological activity of denatured lipoprotein. Evidently, it exerts a very important effect on the commercialization of diagnostic technology and the development of reagents with a view to various objects mentioned above.

## Claims

1. A method for the production of denatured lipoprotein, comprising the steps of:
freezing a solution containing lipoprotein to produce a frozen solution; and
melting the frozen solution to produce a solution containing denatured lipoprotein.

2. A method according to claim 1, wherein the steps of freezing and melting are repeated.

3. A method according to claim 2, wherein the steps of freezing and melting are repeated in the range of 1 to 10 times.

4. A method according to claim 2, wherein the steps of freezing and melting are repeated in the range of 1 to 4 times.

5. A method according to any one of claims 1-4, wherein said denatured lipoprotein is a standard substance for determining denatured lipoprotein in blood or an experimental reagent for investigating the physiological role or the physiological activity of denatured lipoprotein.

6. A method according to any one of claims 1-5, wherein said denatured lipoprotein is capable of reacting with a DLH3 antibody which is yielded by hybridoma cell line, mouse-mouse hybridoma FOH1a/DLD3 (Deposit No. FERM BP-7171).

7. A method according to any of claims 1 to 6, wherein said lipoprotein is at least one selected from the group consisting of chylomicrons, VLDL, LDL, Lp(a), HDL2, and HDL3.

8. A method according to any preceding claim, further comprising freeze-drying the solution containing denatured lipoprotein.

9. A method according to claim 8, further comprising adding a stabilizer to the solution containing denatured lipoprotein after melting and before freeze-drying.

10. Stabilized denatured lipoprotein produced by a method according to any preceding claim.

11. A method for the determination of denatured lipoprotein in a sample, comprising the steps of:
selecting a stabilized denatured lipoprotein as set forth in claim 10 as a standard substance;
forming a calibration curve using prescribed concentrations of the standard substance;
reacting the sample with an antibody which binds to the denatured lipoprotein; and
measuring the reactivity of the antibody with the sample.

12. A method according to claim 11, wherein said determination is an immunological determination.

13. A method according to claim 12, wherein said immunological determination is selected from a radio immunoassay, an enzyme immunoassay, a fluoroimmunoassay, a luminescent immunoassay, an agglutination immunoassay, immunonephelometry, and a nephelometric immunoassay.

14. A method according to claim 13, wherein said method for immunological determination is a competitive method or a sandwich method.

15. A reagent kit for the determination of denatured lipoprotein, comprising stabilized denatured lipoprotein set forth in claim 10 as a standard substance.

16. A reagent kit for the determination of denatured lipoprotein, comprising a diluting liquid for a sample, a solid phase formed by immobilizing an antibody, a reaction buffer, a washing solution, a labelled secondary antibody, a detecting reagent, and the whole or part of stabilized denatured lipoprotein set forth in claim 10 as a standard substance as component elements.

## Patentansprüche

1. Verfahren zur Herstellung von denaturiertem Lipoprotein, bei dem man
eine Lösung, die Lipoprotein enthält, einfriert, um eine gefrorene Lösung herzustellen; und
die gefrorene Lösung schmilzt, um eine Lösung herzustellen, die denaturiertes Lipoprotein enthält.

2. Verfahren nach Anspruch 1, wobei die Schritte des Einfrierens und Schmelzens wiederholt werden.

3. Verfahren nach Anspruch 2, wobei die Schritte des Einfrierens und Schmelzens im Bereich von 1 bis 10mal wiederholt werden.

4. Verfahren nach Anspruch 2, wobei die Schritte des Einfrierens und Schmelzens im Bereich von 1 bis 4mal wiederholt werden.

5. Verfahren nach einem der Ansprüche 1 bis 4, wobei dieses denaturierte Lipoprotein eine Standardsubstanz zur Bestimmung von denaturiertem Lipoprotein in Blut oder ein Untersuchungsreagenz zur Ermittlung der physiologischen Funktion oder der physiologischen Aktivität von denaturiertem Lipoprotein ist.

6. Verfahren nach einem der Ansprüche 1-5, wobei dieses denaturierte Lipoprotein in der Lage ist, mit einem DLH3-Antikörper zu reagieren, der von der Hybridomazelllinie Maus-Maus-Hybridoma FOH1a/DLD3 (Hinterlegungsnummer FERM BP-7171) gewonnen wird.

7. Verfahren nach einem der Ansprüche 1-6, wobei dieses Lipoprotein wenigstens eines ist, das aus der Gruppe ausgewählt wird, die aus Chylomicronen, VLDL, LDL, Lp(a), HDL2 und HDL3 besteht.

8. Verfahren nach einem der vorangegangenen Ansprüche, bei dem man ferner die Lösung, die das denaturierte Lipoprotein enthält, gefriertrocknet.

9. Verfahren nach Anspruch 8, bei dem man ferner nach dem Schmelzen und vor dem Gefriertrocknen einen Stabilisator zur Lösung, die das denaturierte Lipoprotein enthält, zugibt.

10. Stabilisiertes denaturiertes Lipoprotein, das nach einem der vorangegangenen Ansprüche hergestellt worden ist.

11. Verfahren zur Bestimmung von denaturiertem Lipoprotein in einer Probe, bei dem man
ein stabilisiertes denaturiertes Lipoprotein, wie es in Anspruch 10 dargelegt ist, als Standardsubstanz auswählt;
eine Kalibrierungskurve unter Verwendung von vorbestimmten Konzentrationen der Standardsubstanz erstellt;
die Probe mit einem Antikörper reagieren lässt, der an das denaturierte Lipoprotein bindet; und
die Reaktivität des Antikörpers mit der Probe misst.

12. Verfahren nach Anspruch 11, wobei diese Bestimmung eine immunologische Bestimmung ist.

13. Verfahren nach Anspruch 12, wobei diese immunologische Bestimmung ausgewählt wird aus einem Radioimmunassay, einem Enzymimmunassay, einem Fluoroimmunassay, einem Lumineszenzimmunassay, einem Agglutinationsimmunassay, Immunnephelometrie und einem nephelometrischen Immunassay.

14. Verfahren nach Anspruch 13, wobei dieses Verfahren zur immunologischen Bestimmung ein kompetitives Verfahren oder ein Sandwichverfahren ist.

15. Reagenzkit zur Bestimmung von denaturiertem Lipoprotein, umfassend stabilisiertes denaturiertes Lipoprotein, wie es in Anspruch 10 dargelegt ist, als Standardsubstanz.

16. Reagenzkit zur Bestimmung von denaturiertem Lipoprotein, umfassend als Komponentenbestandteile eine Verdünnungsflüssigkeit für eine Probe, eine feste Phase, die durch die Immobilisierung eines Antikörpers gebildet wird, ein Reaktionspuffer, eine Waschlösung, ein markierter sekundärer Antikörper, ein Detektionsreagenz und das ganze oder ein Teil des stabilisierten denaturierten Lipoproteins, wie es in Anspruch 10 dargelegt ist, als Standardsubstanz.

## Revendications

1. Procédé pour la production d'une lipoprotéine dénaturée, comprenant les étapes suivantes:
congélation d'une solution contenant une lipoprotéine dans le but de produire une solution congelée; et
décongélation de la solution congelée pour produire une solution contenant une lipoprotéine dénaturée.

2. Procédé selon la revendication 1, dans lequel on répète les étapes de congélation et de décongélation.

3. Procédé selon la revendication 2, dans lequel on répète les étapes de congélation et de décongélation dans la plage de 1 à 10 fois.

4. Procédé selon la revendication 2, dans lequel on répète les étapes de congélation et de décongélation dans la plage de 1 à 4 fois.

5. Procédé selon l'une quelconque des revendications 1 à 4, dans lequel ladite lipoprotéine dénaturée est une substance standard pour la détermination d'une lipoprotéine dénaturée dans le sang, ou est un réactif expérimental pour étudier le rôle physiologique ou l'activité physiologique de la lipoprotéine dénaturée.

6. Procédé selon l'une quelconque des revendications 1 à 5, dans lequel ladite lipoprotéine dénaturée est capable de réagir avec un anticorps DLH3 qui est produit par une lignée cellulaire d'hybridome, à savoir l'hybridome FOH1a/DLD3 de type souris-souris (déposé sous le numéro FERM BP-7171).

7. Procédé selon l'une quelconque des revendications 1 à 6, dans lequel ladite lipoprotéine est au moins une lipoprotéine choisie dans le groupe constitué des chylomicrons, VLDL, LDL, Lp(a), HDL2 et HDL3.

8. Procédé selon l'une quelconque des revendications précédentes, comprenant une étape de lyophilisation de la solution contenant la lipoprotéine dénaturée.

9. Procédé selon la revendication 8, comprenant également l'addition d'un stabilisateur à la solution contenant la lipoprotéine dénaturée après la décongélation et avant la lyophilisation.

10. Lipoprotéine dénaturée stabilisée produite par un procédé selon l'une quelconque des revendications précédentes.

11. Procédé de détermination d'une lipoprotéine dénaturée dans un échantillon, comprenant les étapes suivantes:
- sélectionner une lipoprotéine dénaturée stabilisée comme présentée dans la revendication 10, comme substance standard;
- former une courbe d'étalonnage en utilisant des concentrations prescrites de la substance standard;
- faire réagir l'échantillon avec un anticorps qui se lie à la lipoprotéine dénaturée; et
- mesurer la réactivité de l'anticorps avec l'échantillon.

12. Procédé selon la revendication 11, dans lequel ladite détermination est une détermination immunologique.

13. Procédé selon la revendication 12, dans lequel ladite détermination immunologique est choisie parmi un essai radio-immunologique, un essai immuno-enzymatique, un essai d'immuno-fluorescence, un essai d'immuno-luminescence, un essai immunologique d'agglutination, l'immunonéphélométrie et un essai immuno-néphélométrique.

14. Procédé selon la revendication 13, dans lequel ledit procédé pour effectuer une détermination immunologique est un procédé compétitif ou un procédé de type sandwich.

15. Trousse de réactifs pour déterminer une lipoprotéine dénaturée, comprenant la lipoprotéine dénaturée stabilisée présentée dans la revendication 10 comme substance standard.

16. Trousse de réactifs pour la détermination d'une lipoprotéine dénaturée, comprenant comme éléments de composition, un liquide de dilution pour un échantillon, une phase solide formée en immobilisant un anticorps, un tampon de réaction, une solution de lavage, un anticorps secondaire marqué, un réactif de détection et l'intégralité ou une partie de la lipoprotéine dénaturée stabilisée présentée dans la revendication 10, comme substance standard.
